(19) Europäisches Patentamt
European Patent Office
Office européen des brevets

(11) EP 2 809 649 B1

(12) **EUROPÄISCHE PATENTSCHRIFT**

(45) Veröffentlichungstag und Bekanntmachung des Hinweises auf die Patenterteilung:
**07.03.2018 Patentblatt 2018/10**

(21) Anmeldenummer: **13701376.9**

(22) Anmeldetag: **10.01.2013**

(51) Int Cl.:
***C07C 309/17*** *(2006.01)* ***C11D 1/29*** *(2006.01)*
***A61K 8/46*** *(2006.01)* ***A61Q 19/10*** *(2006.01)*

(86) Internationale Anmeldenummer:
**PCT/EP2013/050367**

(87) Internationale Veröffentlichungsnummer:
**WO 2013/113536 (08.08.2013 Gazette 2013/32)**

(54) **POLYGLYCOLETHERFREIE SULFOSUCCINATE BASIEREND AUF POLYGLYCERINPARTIALESTERN UND DEREN VERWENDUNG**

POLYGLYCOL-ETHER-FREE SULFOSUCCINATES BASED ON POLYGLYCERIN PARTIAL ESTERS AND USE THEREOF

SULFOSUCCINATES EXEMPTS DE POLYÉTHERS DE GLYCOL ET BASÉS SUR DES ESTERS PARTIELS DE POLYGLYCÉROL AINSI QUE LEUR UTILISATION

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **03.02.2012 DE 102012201598**

(43) Veröffentlichungstag der Anmeldung:
**10.12.2014 Patentblatt 2014/50**

(73) Patentinhaber: **Evonik Degussa GmbH**
**45128 Essen (DE)**

(72) Erfinder:
- **HARTUNG, Christian**
**45133 Essen (DE)**
- **SCHUCH, Dominik**
**42781 Haan (DE)**
- **BERKELS, Wolfgang**
**46238 Bottrop (DE)**
- **MUSS, Peter**
**45359 Essen (DE)**
- **WESTERHOLT, Ursula**
**45138 Essen (DE)**
- **HERRWERTH, Sascha**
**45134 Essen (DE)**
- **PEGGAU, Joerg**
**45357 Essen (DE)**
- **SPRINGER, Oliver**
**46485 Wesel (DE)**
- **WENK, Hans, Henning**
**45470 Mühlheim an der Ruhr (DE)**
- **KLEIN, Ralf**
**42549 Velbert (DE)**

(56) Entgegenhaltungen:
**EP-A2- 0 794 173 WO-A1-92/18470**
**SU-A1- 1 330 130 US-A1- 2006 207 034**

- **DATABASE Chemical Abstracts, Chemi [Online] 10. Dezember 1979 (1979-12-10), XP002044948, gefunden im CHEMICAL Database accession no. 194959s**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 809 649 B1

## Beschreibung

### Gebiet der Erfindung

Gegenstand der Erfindung sind polyglycoletherfreie, auf Polyglycerinpartialestern basierende Sulfosuccinate, deren Herstellung, sowie die Verwendung dieser in kosmetischen Formulierungen sowie in Reinigungsmitteln des Industrie- und Haushaltsbereichs und die diese Sulfosuccinate enthaltenden Formulierungen selber.

### Stand der Technik

Sulfosuccinate sind viel benutzte Sekundärtenside. Sulfosuccinate werden v.a. wegen ihrer guten Hautverträglichkeit (Mildheit) und sehr guten Schaumeigenschaften verwendet, z.B. in Baby Shampoos und in Reinigungsmitteln für empfindliche Haut.

Sulfosuccinate werden maßgeblich als alkoxylierte, insbesondere ethoxylierte, Produkte eingesetzt. Standardprodukt auf dem Markt ist das Disodium Laureth Sulfosuccinate (z.B. erhältlich unter dem Namen "REWOPOL® SB FA 30 B" von Evonik Industries AG). Nicht-ethoxylierte Tenside (z.B. Disodium Lauryl Sulfate oder Disodium Lauryl Sulfosuccinate) haben üblicherweise die Nachteile, dass sie reizendere Eigenschaften im Vergleich zu den entsprechenden alkoxylierten Derivaten haben und häufig Feststoffe sind, ein Umstand, der die Verarbeitbarkeit erschwert.

In WO1992018470 werden Sulfosuccinate auf Basis ethoxylierter Monoglycerylfettsäureester und deren Herstellung und Verwendung in beispielsweise kosmetischen Reinigungsmitteln beschrieben.

In DE102005012479 wird die Anwendung von Sulfosuccinaten auf Basis von Partial-(mono)glyceriden als anionische Weichmacher beansprucht.

In den Schriften EP0794173 und SU1330130 werden Polyglycerinester-basierte Sulfosuccinate und deren Einsatz als Tenside für Reinigungsmittel beschrieben. Die beschriebenen Produkte sind sehr mild und bilden stabile Formulierungen mit gutem Schaumvermögen und Hautgefühl. Die in diesen Schriften beschriebenen Produkte weisen alle einen Veresterungsgrad des Polyglycerins mit der Fettsäure von mindestens 1 auf, das heißt jedes Moläquivalent Polyglycerin wurde mit mindestens einem Moläquivalent Fettsäure verestert. Zur Lagerstabiltät der Sulfosuccinat-Produkte und zur Verdickbarkeit der mit diesen Produkten hergestellten Formulierungen werden hier allerdings keine Aussagen getroffen.

Nachteilig an den im Stand der Technik beschriebenen, vergleichsweise milden Sulfosuccinaten ist, dass es sich dabei ganz überwiegend um alkoxylierte, insbesondere ethoxylierte Produkte handelt. Der generelle Markttrend vor allem in der Kosmetikindustrie geht aber heute zu polyglycoletherfreien Produkten.
Des Weiteren ist das ethoxylierte Standardprodukt Disodium Laureth Sulfosuccinate im RBC-Test (<u>R</u>ed <u>B</u>lood <u>C</u>ell-Test) mit L/D Werten von knapp unter 10 immer noch als mäßig reizend einzustufen. Es werden aber noch mildere und dabei polyglycoletherfreie Produkte vom Markt und den Konsumenten gefordert.
Nachteilig an den in EP0794173 (und SU1330130) beschriebenen auf Polyglycerinestern basierenden Sulfosuccinaten ist, dass sie als konzentrierte wässrige Lösungen mit einem Trockengehalt von mindestens 30% keine klaren, homogenen und lagerstabilen Produkte liefern, sondern trübe Dispersionen bilden, welche rasch in zwei Phasen separieren (siehe Beispiele 2.8 und 2.9). Zudem sind die Tensideigenschaften der wässrigen Lösungen mit Oberflächenspannungen von bis zu einem Minimum von ca. 33 mN/m nur als mittelmäßig einzustufen.

Auch sind alle bisher bekannten, kommerziell erhältlichen wässrigen Sulfosuccinate, einschließlich der ethoxylierten Produkte, in der Regel nicht über längere Zeiträume (d.h. > 12 Monate bei 25 °C) oder nach Gefrier-Tau-Cyclen (Einfrieren auf-15 °C und wieder Auftauen lassen auf 20 °C) lagerstabil. Es bilden sich dann mehrphasige Gemische aus.

Bisher eingesetzte Sulfosuccinate haben zudem grundsätzlich Nachteile bei der Verdickung der mit ihnen hergestellten tensidischen Formulierungen, da sie hohe Mengen an Verdickern erfordern und der Einsatz von NaCl oft nicht möglich ist.

Aufgabe der vorliegenden Erfindung war es, äußerst milde, reinigungsaktive Tenside zur Verfügung zu stellen.

### Beschreibung der Erfindung

Überraschenderweise wurde gefunden, dass die im Folgenden beschriebenen Sulfosuccinate basierend auf Polyglycerinpartialestern die Nachteile aus dem Stand der Technik beheben und damit die der Erfindung gestellte Aufgabe zu lösen vermögen.

Gegenstand der vorliegenden Erfindung sind daher bestimmte, von Polyglycerinpartialestern abgeleitete, polyglycoletherfreie Sulfosuccinate der allgemeinen Formel I, ein Verfahren zur Herstellung von auf Polyglycerinpartialestern basierenden, polyglycoletherfreien Sulfosuccinaten sowie deren Verwendung zur Herstellung von Formulierungen. Die diese erfindungsgemäßen Sulfosuccinate enthaltenden Formulierungen selbst sind ebenfalls Gegenstand der Erfindung.

Ein Vorteil der vorliegenden Erfindung ist es, dass die erfindungsgemäßen Sulfosuccinate ein sehr reduziertes Reizpotential in der Größenordnung von L/D ≥100 in einem RBC-Test aufweisen.
Noch ein Vorteil der erfindungsgemäßen Sulfosuccinate ist es, dass sie eine sehr hohe Oberflächenaktivität aufweisen, so dass ihre wässrigen Lösungen Oberflächenspannungen von <33 mN/m aufweisen.
Ein weiterer Vorteil der erfindungsgemäßen Sulfosuccinate ist es, dass sie polyglycoletherfrei sind.
Noch ein Vorteil der erfindungsgemäßen Sulfosuccinate ist es, dass sie gut verdickbare Formulierungen ergeben. Insbesondere kann in ihnen teilweise auch NaCl als Verdicker eingesetzt werden.
Noch ein Vorteil der erfindungsgemäßen Sulfosuccinate ist es, dass sie gute Schaumeigenschaften aufweisen.
Noch ein Vorteil der erfindungsgemäßen Sulfosuccinate ist es, dass sie klare, homogene wässrige Lösungen ausbilden.
Ein weiterer Vorteil der erfindungsgemäßen Sulfosuccinate ist es, dass sie sich mit einem einfachen Verfahren als hoch konzentrierte, mit einem Trockengehalt von > 55 Gew.-%, wässrige Sulfosuccinat-Tenside darstellen lassen.
Ein weiterer Vorteil der erfindungsgemäßen Sulfosuccinate ist die hohe Lagerstabilität ihrer hoch konzentrierten wässrigen Lösungen und der diese enthaltenden Formulierungen.
Ein weiterer Vorteil ist es, dass die erfindungsgemäßen Sulfosuccinate auf natürlichen, nachwachsenden Rohstoffen beruhen und gut biologisch abbaubar sind.

Noch ein Vorteil ist, dass die erfindungsgemäßen Sulfosuccinate die Emollienteigenschaften der Formulierungen auf der Haut sowie die Pflege- und Reinigungswirkung von Formulierungen für Haushalt und Industrie verstärken.

Gegenstand der vorliegenden Erfindung sind somit auf Polyglycerinpartialestern basierende Sulfosuccinate der allgemeinen Formel I

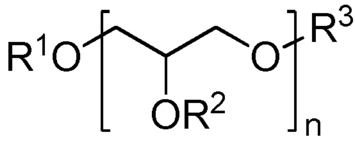 allgemeine Formel I

mit

$R^1$, $R^2$, $R^3$ = unabhängig voneinander gleich oder verschieden H, $R^4$ oder $R^5$,

$R^4$ = gesättigter oder teilweise ungesättigter, 6-22 C-Atome enthaltender Acylrest, der durch Hydroxylgruppen substituiert sein kann, bevorzugt ein Acylrest mit 8 - 18 C-Atomen, insbesondere ein Capryloyl-, Caproyl- oder Lauroylrest, wobei auch Mischungen von Acylresten vorliegen können,

$R^5$ = ausgewählt aus Sulfobernsteinsäurereste der Formel IIa oder IIb

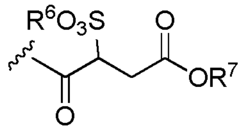 Formel IIa, 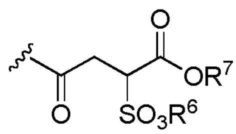 Formel IIb

und Sulfomethylbernsteinsäurereste der Formel IIc oder IId

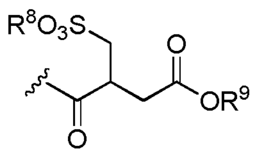 Formel IIc, 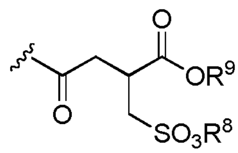 Formel IId,

mit
$R^6$, $R^7$, $R^8$, $R^9$ = unabhängig voneinander gleich oder verschieden H, ein Alkali- oder ½ Erdalkalimetall oder eine Ammoniumgruppe, bevorzugt Natrium,

n = 2 bis 16,

dadurch gekennzeichnet, dass
im statistischen Mittel pro Molekül der allgemeinen Formel I 0,2 bis 0,8, bevorzugt 0,3 bis 0,6 Reste $R^4$ und im statistischen Mittel pro Molekül der allgemeinen Formel I 0,3 bis 6, bevorzugt 0,5 bis 5, insbesondere bevorzugt 0,7 bis 3 Reste $R^5$

vorliegen.

Erfindungsgemäß bevorzugte Sulfosuccinate der allgemeinen Formel I sind dadurch gekennzeichnet, dass sie im statistischen Mittel ein Gewichtsverhältnis von eingesetzter Carbonsäure $R^4OH$ zu eingesetztem Polyglycerin (allgemeine Formel I mit $R^1$, $R^2$, $R^3$ = H) von 0,10 zu 1 bis 0,50 zu 1, insbesondere von 0,15 zu 1 bis 0,40 zu 1 aufweisen.

Dem Fachmann ist bewusst, dass das in der allgemeinen Formel I enthaltene Polyglycerin-Grundgerüst aufgrund seiner polymeren Eigenschaft eine statistische Mischung verschiedener Verbindungen darstellt. Polyglycerin kann ausgebildete Etherbindungen zwischen zwei primären, einer primären und einer sekundären sowie zwei sekundären Positionen der Glycerinmonomere aufweisen. Aus diesem Grund besteht das Polyglycerin-Grundgerüst üblicherweise nicht ausschließlich aus linear verknüpften Glycerin-Einheiten, sondern kann auch Verzweigungen und Cyclen enthalten. Für Details siehe z.B. "Original synthesis of linear, branched and cyclic oligoglycerol Standards", Cassel et al., J. Org. Chem. 2001, 875-896.

Entsprechende Strukturen sind von der in dieser Hinsicht vereinfachten, allgemeinen Formel I miterfasst.

Die Fettsäurereste $R^4$ und die Reste $R^5$ können statistisch an das Polyglyceringrundgerüst sowohl über primäre als auch über sekundäre Hydroxylgruppen gebunden sein.

Der Begriff "Sulfosuccinat" im Zusammenhang mit der vorliegenden Verbindung bezieht sich auf die Bernsteinsäure-basierenden Sulfosuccinate und auf die Itaconsäure-basierenden Sulfomethylsuccinate.

Alle Bedingungen wie beispielsweise Druck und Temperatur sind, wenn nicht anders angegeben, Standardbedingungen (20 °C, 1 bar). Prozentzahlen sind, soweit nicht anders beschrieben, in Massenprozent angegeben.

Erfindungsgemäß bevorzugte Sulfosuccinate der allgemeinen Formel I sind dadurch gekennzeichnet, dass sie einen mittleren Polymerisationsgrad n von 2,5 bis 12, besonders bevorzugt von 3 bis 8 aufweisen.

Der Polymerisationsgrad n lässt sich dadurch bestimmen, dass die Hydroxylzahl des zur Synthese des erfindungsgemäßen Sulfosuccinates eingesetzten Polyglycerins ermittelt wird, wobei der mittlere Polymerisationsgrad n mit der Hydroxylzahl des zugrundeliegenden Polyglycerins über folgende Gleichung verknüpft ist:

$$n = \frac{\dfrac{2000 \bullet M(KOH)}{OHZ} - M(Wasser)}{\left[ \left[ M(Glycerin) - M(Wasser) \right] - \dfrac{1000 \bullet M(KOH)}{OHZ} \right]}$$

mit M = molare Masse; OHZ = Hydroxylzahl des freien Polyglycerins.

Alternativ lässt sich der Polymerisationsgrad n auch dadurch bestimmen, dass die Hydroxylzahl des nach vollständiger Hydrolyse des Sulfosuccinates erhaltenen Polyglycerins ermittelt wird.

Geeignete Bestimmungsmethoden zur Ermittlung der Hydroxylzahl sind insbesondere solche gemäß DGF C-V 17 a (53), Ph. Eur. 2.5.3 Method A und DIN 53240.

Erfindungsgemäß bevorzugte Sulfosuccinate der allgemeinen Formel I sind dadurch gekennzeichnet, dass $R^4$ = den Acylrest einer oder mehrerer natürlichen Fettsäuren mit 8-18 C-Atomen darstellt, insbesondere ein Acylrest der Fettsäuren ausgewählt aus Önanthsäure, Caprylsäure, Pelargonsäure, Caprinsäure, Laurinsäure, Myristinsäure, Pentadecansäure, Palmitinsäure, Margarinsäure, Stearinsäure, Undecylensäure, Myristoleinsäure, Palmitoleinsäure, Petroselinsäure, Ölsäure, Elaidinsäure, Vaccensäure, Linolsäure, $\alpha$-Linolensäure, $\gamma$-Linolensäure, Calendulasäure, Punicinsäure, $\alpha$-Elaeostearinsäure, $\beta$-Elaeostearinsäure und deren Mischungen, wobei Capryl-, Caprin- oder Laurinsäure, insbesondere deren Mischungen, und Kokosfettsäuremischungen besonders bevorzugt sind.

Erfindungsgemäß kann $R^4$ durch Mischungen von entsprechenden Carbonsäuren gebildet werden, insbesondere bevorzugt sind hier Kokosfettsäuremischungen und deren technische Schnitte.

Erfindungsgemäß bevorzugte Sulfosuccinate der allgemeinen Formel I sind dadurch gekennzeichnet, dass $R^5$ ausgewählt ist aus Sulfobernsteinsäureresten der Formel IIa oder IIb.

In einer erfindungsgemäßen alternativen Ausführungsform sind bevorzugte Sulfosuccinate der allgemeinen Formel I, dadurch gekennzeichnet, dass $R^5$ ausgewählt ist aus Sulfomethylbernsteinsäureresten der Formel IIc oder IId.

Erfindungsgemäß besonders bevorzugte Sulfosuccinate der allgemeinen Formel I sind dadurch gekennzeichnet, dass $R^4$ = der Acylrest einer natürlichen Fettsäure ausgewählt aus Caprylsäure, Caprinsäure und Laurinsäure, insbesondere deren Mischungen, und Kokosfettsäuremischungen ist, und

$R^5$ ausgewählt ist aus Sulfobernsteinsäureresten der Formel IIa oder IIb.

Ein weiterer Gegenstand der vorliegenden Erfindung ist ein Verfahren zur Herstellung von auf Polyglycerinpartialestern basierenden Sulfosuccinaten umfassend die Verfahrensschritte:

A) Umsetzen von Polyglycerin mit einem Polymerisierungsgrad von 2 - 16 mit 20 - 80 Molprozent, bevorzugt mit 30 - 60 Molprozent, bezogen auf das Polyglycerin, einer oder mehrere gesättigten oder teilweise ungesättigten, 6-22

C-Atome enthaltender Carbonsäure, die durch Hydroxylgruppen substituiert sein kann, bevorzugt einer Carbonsäure mit 8 - 18 C-Atomen, insbesondere mit einer Caprylsäure, Caprinsäure oder Laurinsäure, insbesondere deren Mischungen, und Kokosfettsäuremischungen,

B) ausgewählt aus mindestens einem der Verfahrensschritte

B1) Umsetzen mit 30 - 600, bevorzugt 50 - 500, insbesondere 70 - 300 Molprozent Maleinsäureanhydrid und/oder Itaconsäureanhydrid, bezogen auf das Polyglycerin, und

B2) Umsetzen mit 30 - 600, bevorzugt 50 - 500, insbesondere 70 - 300 Molprozent Itaconsäure, bezogen auf das Polyglycerin,

C) Sulfonierung mit Alkali-, Erdalkali- und/oder Ammoniumsulfit-Salzen wie Natriumsulfit oder Natriumhydrogensulfit bzw. Natriumdisulfit und gegebenenfalls

D) Aufreinigung der auf Polyglycerinpartialestern basierenden Sulfosuccinate.

Da das erfindungsgemäße Verfahren - bis auf die aufgrund des Polyglycerins enthaltenen Polyetherstrukturen - polyglycoletherfreie Sulfosuccinate bereitstellen soll, sind in dem erfindungsgemäßen Verfahren weitere Verfahrensschritte ausgeschlossen, die zu weiteren Polyetherstrukturen führen könnten; solche ausgenommenen Verfahrensschritte sind insbesondere Alkoxylierungen, wie Ethoxylierungen (mit Ethylenoxid) oder Propoxylierungen (mit Propylenoxid).

Verfahrensschritt A) wird bevorzugt durchgeführt wie in G. Jakobson, Fette, Seifen, Anstrichmittel 1986, 88, 101-105 beschrieben. Insbesondere wird erfindungsgemäß in Verfahrensschritt A) das Polyglycerin mit der oder den Carbonsäuren, gegebenenfalls unter Mitverwendung eines Veresterungskatalysators, bei 150 - 250 °C unter Entfernung des Reaktionswassers verestert.

Verfahrensschritte B) und C) werden bevorzugt durchgeführt wie in DE-OS 27 00 072 beschrieben. Dabei wird in B1) bei 60 - 80 °C bis zur vollständigen Reaktion des Anhydrids gerührt. Verfahrensschritt B2) wird mit Itaconsäure bei einer Temperatur von 120-250 °C durchgeführt, wobei dieser Verfahrensschritt B2) auch mit Verfahrensschritt A als Eintopfsynthese kombinierbar ist. In Verfahrensschritt C) wird der Maleinsäureester und/oder der Itaconsäureester in eine wässrige Natriumsulfit- oder Natriumhydrogensulfit bzw. Natriumdisulfit-Lösung gegeben und bei 60 - 90 °C sulfoniert bis das Sulfit vollständig abreagiert ist.

Optional wird das wässrige Produkt pH-neutral eingestellt.

Erfindungsgemäß bevorzugte Verfahren sind dadurch gekennzeichnet, dass in Verfahrensschritt A) das Polyglycerin mit 10 - 50 Gewichtsprozent, bevorzugt mit 15 - 40 Gewichtsprozent, bezogen auf das Polyglycerin, mindestens einer Carbonsäure umgesetzt wird.

Erfindungsgemäß bevorzugte Verfahren sind dadurch gekennzeichnet, dass in Verfahrensschritt A) das Polyglycerin einen mittlere Polymerisationsgrad n von 2 - 11, besonders bevorzugt von 3 - 8 aufweist, wobei dieser wie oben beschrieben bestimmt werden kann.

In Verfahrensschritt A) werden bevorzugt natürliche Fettsäuren mit 8 - 18 C-Atomen, insbesondere Fettsäuren ausgewählt aus Önanthsäure, Caprylsäure, Pelargonsäure, Caprinsäure, Laurinsäure, Myristinsäure, Pentadecansäure, Palmitinsäure, Margarinsäure, Stearinsäure, Undecylensäure, Myristoleinsäure, Palmitoleinsäure, Petroselinsäure, Ölsäure, Elaidinsäure, Vaccensäure, Linolsäure, $\alpha$-Linolensäure, $\gamma$-Linolensäure, Calendulasäure, Punicinsäure, $\alpha$-Elaeostearinsäure und $\beta$-Elaeostearinsäure, wobei Caprylsäure, Caprinsäure und Laurinsäure, insbesondere deren Mischungen, und Cocosfettsäuren besonders bevorzugt sind, eingesetzt.

In Verfahrensschritt B) wird bevorzugt Maleinsäureanhydrid gemäß B1) eingesetzt.

In einer alternativern Ausführungsform des erfindungsgemäßen Verfahrens wird in Verfahrensschritt B) bevorzugt Itaconsäureanhydrid gemäß B1) oder Itaconsäure gemäß B2) eingesetzt.

Erfindungsgemäß besonders bevorzugte Verfahren setzen in Verfahrensschritt A) mindestens eine Carbonsäure ausgewählt aus Caprylsäure, Caprinsäure und Laurinsäure, insbesondere deren Mischungen, und Kokosfettsäuremischungen und in Verfahrensschritt B) Maleinsäureanhydrid gemäß B1) ein.

Es ist erfindungsgemäß bevorzugt, dass am Ende des Verfahrensschrittes C) eine wässrige Lösung mit einem Trockengehalt von mindestens 40 Gew.-%, insbesondere mindestens 55 Gew.-% bezogen auf den gesamten Reaktionsansatz vorliegt, welches durch Wahl des Wassergehaltes in Verfahrensschritt C) gesteuert werden kann.

Bei Bedarf kann im Rahmen des Verfahrensschrittes D) weiteres Wasser abdestilliert werden. Insbesondere kann hier das Wasser so weit entfernt werden, dass ein wasserfreies Pulver erhalten wird.

Dies kann auch durch andere Trocknungsverfahren wie beispielsweise Sprühtrocknung erreicht werden.

Dieses Pulver kann vorteilhaft gelagert und ebenso vorteilhaft in festen Endprodukten wie zum Beispiel Syndetseifen eingesetzt werden.

Ein weiterer Gegenstand der vorliegenden Erfindung sind auf Polyglycerinpartialestern basierende Sulfosuccinate erhältlich nach dem erfindungsgemäßen Verfahren, wobei erfindungsgemäß Sulfosuccinate besonders bevorzugt sind, die durch erfindungsgemäß bevorzugte Verfahren erhältlich sind.

Die Sulfosuccinate der vorliegenden Erfindung lassen sich vorteilhaft zur Herstellung von Pflege- und Reinigungsformulierungen, insbesondere für Haut und Hautanhangsgebilden, wie beispielsweise Flüssigseifen, Duschgels oder Shampoos, sowie von Pflege- und Reinigungsformulierungen für Haushalt und Industrie verwenden, wobei diese bevorzugt ausgewählt sind aus der Gruppe der kosmetischen, reinigenden und pflegenden Formulierungen. Somit sind derartige Verwendungen ebenfalls Gegenstand der vorliegenden Erfindung.

Unter dem Begriff "Pflegeformulierung" wird hier eine Formulierung verstanden, die den Zweck erfüllt, einen Gegenstand in seiner ursprünglichen Form zu erhalten, die Auswirkungen äußerer Einflüsse (z.B. Zeit, Licht, Temperatur, Druck, Verschmutzung, chemische Reaktion mit anderen, mit dem Gegenstand in Kontakt tretenden reaktiven Verbindungen) wie beispielsweise Altern, Verschmutzen, Materialermüdung, Ausbleichen, zu mindern oder zu vermeiden oder sogar gewünschte positive Eigenschaften des Gegenstandes zu verbessern. Für letzten Punkt sei etwa ein Glanz des betrachteten Gegenstandes genannt.

In diesem Zusammenhang sind die Pflege- und Reinigungsformulierungen nicht auf kosmetische, pharmazeutische oder dermatologische Formulierungen wie z. B. zur Behandlung der Haut und der Haare in Form von Duschgels, Schaumbäder, Flüssigseifen, Haarshampoos, 2in1-Shampoos, Haarspülungen, Dauerwell-Fixierlösungen, Haarfärbeshampoos, Haarfestiger, Haarkuren, Haarlegemittel, Haarstyling-Zubereitungen, Fön-Lotionen, Schaumfestiger, Haarkuren, Leave-In Conditionierer, Haarglättungsmitteln, Glanzverbesserungsmitteln und Mittel zum Färben der Haare sowie anderen reinigenden und pflegenden Formulierungen beschränkt, sondern können auch solche Formulierungen sein, wie man sie in Haushalt und Industrie einsetzt, etwa zur Pflege und Reinigung von Oberflächen nicht lebender Gegenstände wie beispielsweise Fliesen, Holz, Glas, Keramik, Linoleum, Kunststoff, lackierte Oberflächen, Leder, Stoffe, Fasern. Beispiele solcher Gegenstände sind Fensterscheiben und -bänke, Duschabtrennungen, Fußböden wie Teppiche, Fliesen, Laminate, Parkett, Korkfußböden, Marmor-, Stein- und Feinsteinzeugböden, Haushaltskeramiken wie WCs, Waschbecken, Bidets, Duschtassen, Badewannen, Türklinken, Armaturen, Haushaltswerkzeuge wie Waschmaschinen, Trockner, Spülmaschinen, Spülen aus Keramik oder Edelstahl, Möbel wie Tische, Stühle, Regale, Ablageflächen, Fenster, Kochgeschirr, Geschirr und Besteck, Wäsche, insbesondere körpernahe Wäsche ("Unterwäsche"), Wasserfahr-, Fahr- und Flugzeuge wie Autos, Busse, Motor- und Segelboote, Werkzeuge wie chirurgische Instrumente, Staubsauger, Maschinen, Rohrleitungen, Tanks und Geräte für Transport, Verarbeitung und Aufbewahrung in der Lebensmittelverarbeitung. Somit handelt es sich in diesem Zusammenhang um die Verwendung in Reinigungs- und Pflegemitteln für Haushalt, industrielle und institutionelle Gewerbe.

In diesem Zusammenhang handelt es sich bei der zu pflegenden und reinigenden Oberfläche bevorzugt um Kochgeschirr und Haushaltswerkzeuge.

Noch ein Gegenstand der vorliegenden Erfindung sind somit auch die Pflege- und Reinigungsformulierungen, insbesondere für Haut und Hautanhangsgebilden, sowie für Haushalt und Industrie, insbesondere kosmetische Formulierungen, wobei diese bevorzugt ausgewählt sind aus der Gruppe der Haut- und Haarbehandlungsmittel, beispielsweise Shampoos mit oder ohne ausgeprägter Konditionierwirkung, Flüssigseifen und Duschgels, enthaltend erfindungsgemäße Sulfosuccinate.

Eine erfindungemäß bevorzugte Formulierung enthält die erfindungsgemäßen Sulfosuccinate in einer Menge von 0,1 Gew.-% bis 99 Gew.-%, bevorzugt in einer Menge von 0,5 Gew.-% bis 20 Gew.-%, besonders bevorzugt in einer Menge von 1,0 Gew.-% bis 10 Gew.-%, wobei sich die Gew.-% auf die Gesamtformulierung beziehen.

Erfindungsgemäße kosmetische Pflege- und Reinigungsformulierungen, können zum Beispiel mindestens eine zusätzliche Komponente enthalten, ausgewählt aus der Gruppe der

Emollients,
Emulgatoren,
Verdicker/Viskositätsregler/Stabilisatoren,
Antioxidantien,
Hydrotrope (oder Polyole),
Fest- und Füllstoffe,
Perlglanzadditive,
Deodorant- und Antitranspirantwirkstoffe,
Insektrepellentien,
Selbstbräuner,
Konservierungsstoffe,
Konditioniermittel,
Parfüme,
Farbstoffe,
kosmetische Wirkstoffe,
Pflegeadditive,
Überfettungsmittel,
Lösungsmittel.

Substanzen, die als beispielhafte Vertreter der einzelnen Gruppen eingesetzt werden können, sind dem Fachmann bekannt und können beispielsweise der EP2273966A1 entnommen werden. Diese Patentanmeldung wird hiermit als Referenz eingeführt und gilt somit als Teil der Offenbarung.
Bezüglich weiterer fakultativer Komponenten sowie der eingesetzten Mengen dieser Komponenten wird ausdrücklich auf die dem Fachmann bekannten einschlägigen Handbücher, zum Beispiel K. Schrader, "Grundlagen und Rezepturen der Kosmetika", 2. Auflage, Seite 329 bis 341, Hüthig Buch Verlag Heidelberg, verwiesen.
Die Mengen der jeweiligen Zusätze richten sich nach der beabsichtigten Verwendung. Typische Rahmenrezepturen für die jeweiligen Anwendungen sind bekannter Stand der Technik und sind beispielsweise in den Broschüren der Hersteller der jeweiligen Grund- und Wirkstoffe enthalten. Diese bestehenden Formulierungen können in der Regel unverändert übernommen werden. Im Bedarfsfall können zur Anpassung und Optimierung die gewünschten Modifizierungen aber durch einfache Versuche komplikationslos vorgenommen werden.

Bevorzugte erfindungsgemäße Formulierungen sind wässrige, tensidische Formulierungen; solche enthalten mindestens 50 Gew.-%, bevorzugt 70 Gew.-% Wasser und neben den erfindungsgemäßen Sulfosuccinaten mindestens ein weiteres Tensid.
In den erfindungsgemäßen Formulierungen sind neben den erfindungsgemäßen Sulfosuccinaten insbesondere nichtionische Tenside der Komponente ausgewählt aus der Gruppe bestehend aus Glycerinmono- und -diester und Sorbitanmono- und - diester von gesättigten und ungesättigten Fettsäuren, Alkylmono- und -oligoglycoside, Partialester auf Basis linearer, verzweigter, ungesättigter bzw. gesättigter Fettsäuren, Ricinolsäure sowie 12-Hydroxystearinsäure und Glycerin, Polyglycerin, Pentaerythrit, Dipentaerythrit, Zuckeralkohole (z.B. Sorbit), Alkylglucoside (z.B. Methylglucosid, Butylglucosid, Laurylglucosid, Cocoglucosid) sowie Polyglucoside (z.B. Cellulose), Mono-, Di- und Trialkylphosphate und deren Salze, Zitronensäureester wie z.B. Glyceryl Stearate Citrate, Glyceryl Oleate Citrate und Dilauryl Citrate sowie Glyceryl Caprylate, Polyglycerylcaprylate, Polyglycerylcaprate und Mischungen dieser Tenside enthalten.

Insbesondere kosmetische Pflege- und Reinigungsformulierungen sind bevorzugt, die im Wesentlichen polyglycoletherfrei und im Wesentlichen frei von alkoxylierten Verbindungen sind. Unter dem Begriff "im Wesentlichen frei von alkoxylierten Verbindungen" und "im Wesentlichen polyglycoletherfrei" im Zusammenhang mit der vorliegenden Erfindung ist zu verstehen, dass die Formulierungen keine nennenswerten Mengen an alkoxylierten oder polyglycolether enthaltende Verbindungen aufweisen, die eine oberflächenaktive Wirkung ausüben. Insbesondere ist hierunter zu verstehen, dass diese Verbindungen in Mengen von kleiner 1 Gew.-%, bevorzugt von kleiner 0,1 Gew.-%, besonders bevorzugt von kleiner 0,01 Gew.-% bezogen auf die Gesamtformulierung, insbesondere keine nachweisbaren Mengen, enthalten sind.

Insbesondere kosmetische Pflege- und Reinigungsformulierungen sind bevorzugt, die im Wesentlichen sulfatfrei sind.
Unter dem Begriff "im Wesentlichen sulfatfrei" im Zusammenhang mit der vorliegenden Erfindung ist zu verstehen, dass die Formulierungen keine nennenswerten Mengen an sulfatierten organischen Verbindungen aufweisen, die eine oberflächenaktive Wirkung ausüben. Insbesondere ist hierunter zu verstehen, dass sulfatierte organische Verbindungen in Mengen von kleiner 1 Gew.-%, bevorzugt von kleiner 0,1 Gew.-%, besonders bevorzugt von kleiner 0,01 Gew.-% bezogen auf die Gesamtformulierung, insbesondere keine nachweisbaren Mengen, enthalten sind. Beispiele für sulfathaltige Tenside stellen Sodium Laureth Sulfate, Sodium Lauryl Sulfate, Ammonium Laureth Sulfate, Ammonium Lauryl Sulfate, Sodium Myreth Sulfate, Sodium Coco Sulfate, Sodium Trideceth Sulfate oder MIPA-Laureth Sulfate dar.
Ganz besonders bevorzugte kosmetische Pflege- und Reinigungsformulierungen sind im Wesentlichen sulfatfrei und im Wesentlichen polyglycoletherfrei und im Wesentlichen frei von alkoxylierten Verbindungen.

Bevorzugte erfindungsgemäße Reinigungs- und Pflegeformulierungen für Haushalt und Industrie sind textilweichmachende Formulierungen und textilpflegende Wasch- oder Reinigungsmittel, Geschirrspülmittel, Haushaltsreiniger, Desinfektionsmittel, Desinfektionsreiniger, Schaumreiniger, Fußbodenreiniger, Teppichreiniger, Polsterreiniger, Fußbodenpflegeprodukte, Marmorreiniger, Parkettreiniger, Stein- und Keramikbodenreiniger, Wischpflegemittel, Edelstahlreiniger, Glasreiniger, Geschirrspülmittel, Kunststoffreiniger, Sanitärreiniger, Holzreiniger, Lederreiniger, Waschmittel, Wäschepflegemittel Desinfektionswaschmittel, Vollwaschmittel, Feinwaschmittel, Wollwaschmittel, Weichspülmittel und Imprägniermittel, wobei Geschirrspülmittel und Haushaltsreinigungsmitteln, insbesondere Handgeschirrspülmittel besonders bevorzugt sind.

Besonders bevorzugte erfindungsgemäße Reinigungs- und Pflegeformulierungen für Haushalt und Industrie enthalten zusätzlich einen oder mehrere Stoffe aus der Gruppe der Tenside, Gerüststoffe, Bleichmittel, Bleichaktivatoren, Enzyme, Parfüme, Parfümträger, Fluoreszenzmittel, Farbstoffe, Schauminhibitoren, Silikonöle, Antiredepositionsmittel, optische Aufheller, Vergrauungsinhibitoren, Einlaufverhinderer, Knitterschutzmittel, Farbübertragungsinhibitoren, antimikrobiellen Wirkstoffe, Germizide, Fungizide, Antioxidantien, Konservierungsmittel, Korrosionsinhibitoren, Antistatika, Bittermittel, Bügelhilfsmittel, Phobier- und Imprägniermittel, Quell- und Schiebefestmittel, neutrale Füllsalze sowie UV-Absorber.
Insbesondere können die erfindungsgemäßen Reinigungs- und Pflegeformulierungen für Haushalt und Industrie zwischen 0,001 und 90 Gew.-%, besonders bevorzugt 0,01 bis 45 Gew.-% eines oder mehrerer der hier genannten weiteren

Inhaltsstoffe enthalten, wobei sich die Gew.-% auf die gesamte Formulierung beziehen.
Beispiele für einsetzbare Tenside werden in der WO 2007/115872, Seite 17, Zeile 28 bis Seite 21, Zeile 24 beschrieben.
Beispiele für Gerüststoffe, Builder, Bleichmittel, Bleichaktivatoren, Bleichkatalysatoren und Enzyme werden in der WO 2007/115872, Seite 22, Zeile 7 bis Seite 25, Zeile 26 beschrieben.
Antiredepositionsmittel, optische Aufheller, Vergrauungsinhibitoren, Farbübertragungsinhibitoren werden beispielhaft in der WO 2007/115872 auf Seite 26, Zeile 15 bis Seite 28, Zeile 2 beschrieben.
Beispiele von Knitterschutzmitteln, antimikrobiellen Wirkstoffen, Germiziden, Fungiziden, Antioxidantien, Konservierungsmitteln, Antistatika, Bügelhilfsmitteln, UV-Absorbern werden in der WO 2007/115872 auf den Seiten 28, Zeile 14 bis Seite 30, Zeile 22 beschrieben. Deren diesbezüglicher expliziter Offenbarungsgehalt wird durch diese Bezugnahme Teil dieser Offenbarung.

Ein weiteres Anwendungsgebiet, in denen die erfindungsgemäßen Sulfosuccinate vorteilhaft eingesetzt werden können, ist der Pflanzenschutz, da die erfindungsgemäßen Sulfosuccinate dort als Adjuvant wie beispielsweise als Netzmittel fungieren. Somit ist ein weiterer Gegenstand der vorliegenden Erfindung die Verwendung der erfindungsgemäßen Sulfosuccinate zur Herstellung von Pflanzenschutzformulierungen, die Verwendung der erfindungsgemäßen Sulfosuccinate als Adjuvant für Agroanwendungen sowie als Formulierungsadditiv von Pestizidformulierungen.

Die Anwesenheit der erfindungsgemäßen Sulfosuccinate reduziert in diesem Zusammenhang vorteilhaft die notwendige Menge an Aktivwirkstoffen.
Somit ist ein weiterer Gegenstand der vorliegenden Erfindung eine Pflanzenschutzformulierung, welche die erfindungsgemäßen Sulfosuccinate enthält. Bevorzugte Pflanzenschutzformulierungen enthalten neben den erfindungsgemäßen Sulfosuccinate mindestens ein Pestizid.
Die erfindungsgemäßen Sulfosuccinate sowie die Pflanzenschutzformulierungen enthaltend die erfindungsgemäßen Sulfosuccinate lassen sich vorteilhaft zur Oberflächenbehandlung von Grünflächen, wie beispielsweise Golfplätzen, von Torf, Düngemitteln und dergleichen verwenden.

In den nachfolgend aufgeführten Beispielen wird die vorliegende Erfindung beispielhaft beschrieben, ohne dass die Erfindung, deren Anwendungsbreite sich aus der gesamten Beschreibung und den Ansprüchen ergibt, auf die in den Beispielen genannten Ausführungsformen beschränkt sein soll.

Beispiele:

*1. Synthese von Polyglycerinpartialestern als Rohstoffe für die Sulfosuccinatsynthesen:*

*1.1. Synthese von Polyglycerylcaprylat/caprat A:*

Unter Einleiten von Stickstoff wurden 280 g Polyglycerin (Hydroxylzahl = 1104 mg KOH / g) und 70,0 g Capryl/Caprinsäure (Säurezahl = 361 mg KOH / g) bei 240 °C gerührt bis eine Säurezahl von 0,5 mg KOH / g erreicht war. Das im Verlauf der Reaktion gebildete Wasser wurde kontinuierlich abdestilliert. Nach Abkühlung auf Raumtemperatur lag das Reaktionsprodukt als klare Flüssigkeit vor.

*1.2. Synthese von Polyglycerylcaprylat/caprat B:*

Unter Einleiten von Stickstoff wurden 144 g Polyglycerin (Hydroxylzahl = 1113 mg KOH / g) und 36,0 g Capryl/Caprinsäure (Säurezahl = 355 mg KOH / g) bei 210 °C gerührt bis eine Säurezahl von 1,0 mg KOH / g erreicht war. Das im Verlauf der Reaktion gebildete Wasser wurde kontinuierlich abdestilliert. Nach Abkühlung auf Raumtemperatur lag das Reaktionsprodukt als klare Flüssigkeit vor.

*1.3. Synthese von Polyglycerylcaprat:*

Unter Einleiten von Stickstoff wurden 969 g Polyglycerin (Hydroxylzahl = 1020 mg KOH / g) und 236 g Caprinsäure bei 240 °C gerührt bis eine Säurezahl von 0,9 mg KOH / g erreicht war. Das im Verlauf der Reaktion gebildete Wasser wurde kontinuierlich abdestilliert. Nach Abkühlung auf Raumtemperatur lag das Reaktionsprodukt als klare Flüssigkeit vor.

*1.4. Synthese von Polyglycerylcocoat A:*

Unter Einleiten von Stickstoff wurden 137 g Polyglycerin (Hydroxylzahl = 930 mg KOH / g) und 24,2 g raffinierte Kokosfettsäure (C8 - C18; Säurezahl = 270 mg KOH / g) bei 230 °C gerührt bis eine Säurezahl von 0,7 mg KOH / g erreicht war. Das im Verlauf der Reaktion gebildete Wasser wurde kontinuierlich abdestilliert. Nach Abkühlung auf Raumtemperatur lag das Reaktionsprodukt als klare Flüssigkeit vor.

*1.5. Synthese von Polyglyceryllaurat:*

Unter Einleiten von Stickstoff wurden 130 g Polyglycerin (Hydroxylzahl = 960 mg KOH / g) und 22,9 g Laurinsäure bei 240 °C gerührt bis eine Säurezahl von 0,9 mg KOH / g erreicht war. Das im Verlauf der Reaktion gebildete Wasser wurde kontinuierlich abdestilliert. Nach Abkühlung auf Raumtemperatur lag das Reaktionsprodukt als klare Flüssigkeit vor.

*1.6. Synthese von Polyglycerylcocoat B:*

Die Synthese erfolgte entsprechend Beispiel 1.1 aus EP0794173 durch Umsetzung von 229 g Polyglycerin (Hydroxylzahl = 1104 mg KOH / g) mit 210 g gehärteter Kokosfettsäure (C12 - C18; Säurezahl = 257 mg KOH / g). Nach Abkühlung auf Raumtemperatur lag das Reaktionsprodukt als trübe Flüssigkeit vor und wies eine Säurezahl von 2,0 mg KOH / g auf.

*1.7. Synthese von Polyglycerylcocoat C:*

Die Synthese erfolgte entsprechend Beispiel 1.4 aus EP0794173 durch Umsetzung von 87,0 g Polyglycerin-T (Fa. Solvay; Hydroxylzahl = 1060 mg KOH / g) mit 82,7 g raffinierter Kokosfettsäure (C8 - C18; Säurezahl = 270 mg KOH / g). Nach Abkühlung auf Raumtemperatur lag das Reaktionsprodukt als trübe Flüssigkeit vor und wies eine Säurezahl von 0,3 mg KOH / g auf.

*2. Sulfosuccinatsynthesen:*

*2.1. Sulfosuccinat 1 (erfindungsgemäß):*

Entsprechend EP0794173 (Beispiele 3.3 - 3.6) wurden 135 g Polyglycerylcaprylat/caprat A aus Beispiel 1.1 mit 45,1 g Maleinsäureanhydrid umgesetzt und anschließend 94,4 g des erhaltenen Maleinsäureesters mit einer Lösung von 22,3 g Natriumdisulfit in 70,0 g Wasser sulfoniert. Nach Einstellen des pH-Wertes auf 6,5 - 7 mit Natronlauge wurde das Reaktionsprodukt als niederviskose, klare, 60%-ige wässrige Lösung erhalten, die für mindestens 8 Wochen bei Temperaturen zwischen 5 und 50 °C augenscheinlich lagerstabil war und nach 5 Gefrier-Tau-Cyclen (Einfrieren auf-15 °C und wieder Auftauen lassen) äußerlich unverändert, klar und homogen vorlag.

*2.2. Sulfosuccinat 2 (erfindungsgemäß):*

Entsprechend EP0794173 (Beispiele 3.3 - 3.6) wurden 100 g Polyglycerylcaprylat/caprat B aus Beispiel 1.2 mit 30,6 g Maleinsäureanhydrid umgesetzt und anschließend 90,7 g des erhaltenen Maleinsäureesters mit einer Lösung von 20,6 g Natriumdisulfit in 67,0 g Wasser sulfoniert. Nach Einstellen des pH-Wertes auf 6,5 - 7 mit Natronlauge wurde das Reaktionsprodukt als niederviskose, klare, 60%-ige wässrige Lösung erhalten, die für mindestens 8 Wochen bei Temperaturen zwischen 5 und 50 °C augenscheinlich lagerstabil war und nach 5 Gefrier-Tau-Cyclen (Einfrieren auf-15 °C und wieder Auftauen lassen) äußerlich unverändert, klar und homogen vorlag.

*2.3. Sulfosuccinat 3 (erfindungsgemäß):*

Entsprechend EP0794173 (Beispiele 3.3 - 3.6) wurden 423 g Polyglycerylcaprat aus Beispiel 1.3 mit 98,1 g Maleinsäureanhydrid umgesetzt und anschließend 450 g des erhaltenen Maleinsäureesters mit einer Lösung von 82,3 g Natriumdisulfit in 337 g Wasser sulfoniert. Nach Einstellen des pH-Wertes auf 6,5 - 7 mit Natronlauge wurde das Reaktionsprodukt als niederviskose, klare, 60%-ige wässrige Lösung erhalten, die für mindestens 8 Wochen bei Temperaturen zwischen 5 und 50 °C augenscheinlich lagerstabil war und nach 5 Gefrier-Tau-Cyclen (Einfrieren auf-15 °C und wieder Auftauen lassen) äußerlich unverändert, klar und homogen vorlag.

*2.4. Sulfosuccinat 4 (erfindungsgemäß):*

Entsprechend EP0794173 (Beispiele 3.3 - 3.6) wurden 342 g Polyglycerylcaprat aus Beispiel 1.3 mit 158 g Maleinsäureanhydrid umgesetzt und anschließend 170 g des erhaltenen Maleinsäureesters mit einer Lösung von 52,2 g Natriumdisulfit in 136 g Wasser sulfoniert. Nach Einstellen des pH-Wertes auf 6,5 - 7 mit Natronlauge wurde das Reaktionsprodukt als niederviskose, klare, 60%-ige wässrige Lösung erhalten, die für mindestens 8 Wochen bei Temperaturen zwischen 5 und 50 °C augenscheinlich lagerstabil war und nach 5 Gefrier-Tau-Cyclen (Einfrieren auf-15 °C und wieder Auftauen lassen) äußerlich unverändert, klar und homogen vorlag.

*2.5. Sulfosuccinat 5 (erfindungsgemäß):*

Entsprechend EP0794173 (Beispiele 3.3 - 3.6) wurden 295 g Polyglycerylcaprat aus Beispiel 1.3 mit 205 g Maleinsäureanhydrid umgesetzt und anschließend 150 g des erhaltenen Maleinsäureesters mit einer Lösung von 59,6 g Natriumdisulfit in 127 g Wasser sulfoniert. Nach Einstellen des pH-Wertes auf 6,5 - 7 mit Natronlauge wurde das Reaktionsprodukt als niederviskose, klare, 60%-ige wässrige Lösung erhalten, die für mindestens 8 Wochen bei Temperaturen zwischen 5 und 50 °C augenscheinlich lagerstabil war und nach 5 Gefrier-Tau-Cyclen (Einfrieren auf-15 °C und wieder Auftauen lassen) äußerlich unverändert, klar und homogen vorlag.

*2.6. Sulfosuccinat 6 (erfindungsgemäß):*

Entsprechend EP0794173 (Beispiele 3.3 - 3.6) wurden 156 g Polyglycerylcocoat A aus Beispiel 1.4 mit 28,9 g Maleinsäureanhydrid umgesetzt und anschließend 103,8 g des erhaltenen Maleinsäureesters mit einer Lösung von 15,6 g Natriumdisulfit in 74,2 g Wasser sulfoniert. Nach Einstellen des pH-Wertes auf 6,5 - 7 mit Natronlauge wurde das Reaktionsprodukt als niederviskose, klare, 60%-ige wässrige Lösung erhalten, die für mindestens 8 Wochen bei Temperaturen zwischen 5 und 50 °C augenscheinlich lagerstabil war und nach 5 Gefrier-Tau-Cyclen (Einfrieren auf-15 °C und wieder Auftauen lassen) äußerlich unverändert, klar und homogen vorlag.

*2.7. Sulfosuccinat 7 (erfindungsgemäß):*

Entsprechend EP0794173 (Beispiele 3.3 - 3.6) wurden 150 g Polyglyceryllaurat aus Beispiel 1.5 mit 27,4 g Maleinsäureanhydrid umgesetzt und anschließend 75,4 g des erhaltenen Maleinsäureesters mit einer Lösung von 11,3 g Natriumdisulfit in 75,4 g Wasser sulfoniert. Nach Einstellen des pH-Wertes auf 6,5 - 7 mit Natronlauge wurde das Reaktionsprodukt als niederviskose, klare, 60%-ige wässrige Lösung erhalten, die für mindestens 8 Wochen bei Temperaturen zwischen 5 und 50 °C augenscheinlich lagerstabil war und nach 5 Gefrier-Tau-Cyclen (Einfrieren auf -15 °C und wieder Auftauen lassen) äußerlich unverändert, klar und homogen vorlag.

*2.8. Sulfosuccinat 8 (nicht erfindungsgemäß):*

Entsprechend Beispiel 3.4 aus EP0794173 wurden 156 g Polyglycerylcocoat B aus Beispiel 1.6 mit 44,5 g Maleinsäureanhydrid umgesetzt und anschließend 67,2 g des erhaltenen Maleinsäureesters mit einer Lösung von 14,8 g Natriumdisulfit in 122 g Wasser sulfoniert. Der pH-Wert wurde mit Natronlauge eingestellt. Das Reaktionsprodukt wurde als trübe, 40%-ige wässrige Suspension erhalten und separierte nach 2 Tagen bei Raumtemperatur in 2 Phasen.

*2.9. Sulfosuccinat 9 (nicht erfindungsgemäß):*

Entsprechend Beispiel 3.5 aus EP0794173 wurden 79,1 g Polyglycerylcocoat C aus Beispiel 1.7 mit 23,3 g Maleinsäureanhydrid umgesetzt und anschließend 60,0 g des erhaltenen Maleinsäureesters mit einer Lösung von 13,5 g Natriumdisulfit in 109 g Wasser sulfoniert. Der pH-Wert wurde mit Natronlauge eingestellt. Das Reaktionsprodukt wurde als trübe, 40%-ige wässrige Suspension erhalten und separierte nach 2 Tagen bei Raumtemperatur in 2 Phasen.

*2.10. Sulfosuccinat 10 (nicht erfindungsgemäß):*

Polyglycoletherhaltiges Sulfosuccinat REWOPOL® SB FA 30 B (kommerziell erhältlich bei Evonik Industries AG, INCI: Disodium Laureth Sulfosuccinate). Das Produkt besteht aus 40% Sulfosuccinat Aktivwirkstoff.

*2.11. Sulfomethylsuccinat 11 (erfindungsgemäß):*

Entsprechend EP0794173 (Beispiele 3.3 - 3.6) wurden 164 g Polyglycerylcaprylat/caprat B aus Beispiel 1.2 mit 66,0 g Itaconsäureanhydrid umgesetzt und anschließend 94,9 g des erhaltenen Itaconsäureesters mit einer Lösung von 21,9 g Natriumdisulfit in 70,2 g Wasser sulfoniert. Nach Einstellen des pH-Wertes auf 6,5 - 7 mit Natronlauge wurde das Reaktionsprodukt als niederviskose, klare, 60%-ige wässrige Lösung erhalten, die für mindestens 8 Wochen bei 20 °C augenscheinlich lagerstabil war.

*2.12. Sulfomethylsuccinat 12 (erfindungsgemäß):*

Entsprechend EP0794173 (Beispiele 3.3 - 3.6) wurden 172 g Polyglycerylcaprat aus Beispiel 1.3 mit 48,0 g Itaconsäureanhydrid umgesetzt und anschließend 102 g des erhaltenen Itaconsäureesters mit einer Lösung von 17,7

g Natriumdisulfit in 72,8 g Wasser sulfoniert. Nach Einstellen des pH-Wertes auf 6,5 - 7 mit Natronlauge wurde das Reaktionsprodukt als niederviskose, klare, 60%-ige wässrige Lösung erhalten, die für mindestens 8 Wochen bei 20 °C augenscheinlich lagerstabil war.

Tab. 0: Oberflächenspannung (OFS) und RBC-Test ausgewählter Produkte.

| | OFS bei c = 1 g/l [mN/m] | RBC-Test [L/D-Wert] |
|---|---|---|
| Sulfosuccinat 1 | 25,4 | > 100 |
| Sulfosuccinat 3 | 25,4 | > 100 |
| Sulfosuccinat 6 | 31,4 | > 100 |
| Beispiel 3.4 aus EP0794173 | 34,8 | > 100 |
| Beispiel 3.5 aus EP0794173 | 38,4 | > 100 |

Analysenmethoden:

- Oberflächenspannung:

Gemessen mit Krüss Tensiometer K100 bei c = 1 g/l in deionisiertem Wasser.

- Hautverträglichkeit:

RBC-Test, siehe W.J. W. Pape, U. Pfannenbecker, U. Hoppe, Mol. Toxicol. 1987, 1, 525.

| Bewertung: | |
|---|---|
| L/D-Wert | Einstufung |
| >100 | Nicht reizend |
| >10 | Leicht reizend |
| >1 | Mäßig reizend |
| >0,1 | reizend |
| <0,1 | Stark reizend |

- Viskositäten:

Gemessen mit einem Brookfield-Rotationsviskosimeter (Becherglas und Spindel) bei 25 °C nach den Angaben des Geräteherstellers.

*Anwendungstechnische Eigenschaften:*

*Anwendungstechnik Kosmetik:*

Im Folgenden wurden die oben beschriebenen Produkte in kosmetischen Formulierungen ausgetestet. Die Formulierungsbestandteile sind in den Zusammensetzungen in Form der allgemein anerkannten INCI-Nomenklatur unter Verwendung der englischen Begriffe benannt. Alle Konzentrationen in den Anwendungsbeispielen sind in Gewichtsprozent angegeben.

*Austestung der Hautpflegeleistung und der Schaumeigenschaften in Tensidmischungen mittels eines Handwaschtests:*

Zur Bewertung der Hautpflegeleistung und der Schaumeigenschaften der erfindungsgemäßen Sulfosuccinate 1, 3 und 6 sowie des Sulfomethylsuccinats 11 in wässrigen, tensidischen Formulierungen wurden sensorische Handwaschtests im Vergleich zu den Vergleichsbeispielen Sulfosuccinat 8 bzw. 9 und 10 nach dem Stand der Technik durchgeführt. Das Vergleichsbeispiel 10 ist in der Industrie als mildes Sulfosuccinat weit verbreitet und gilt als mildes und universell einsetzbares Tensid für wässrige Formulierungen.

Eine Gruppe bestehend aus 10 trainierten Prüfpersonen wusch sich dabei definiert die Hände und bewertete Schaumeigenschaften und Hautgefühl anhand einer Notenskala von 1 (schlecht) bis 5 (sehr gut).

Die eingesetzten Produkte wurden jeweils in einer standardisierten Tensidformulierung getestet. In einem ersten Test wurde eine Testformulierung mit dem StandardTensidsystem 9% aktiv Sodium Laureth Sulfate und 3% aktiv Sulfosuccinat-Sekundärtensid verwendet (Tabelle 1).

Tab. 1: Testformulierungen für Handwaschtest I:

| Formulierungsbeispiele | 1a | 3a | 6a | 11a | V8a | V10a |
|---|---|---|---|---|---|---|
| Texapon® NSO, BASF Cognis, 28%-ig, (INCI: Sodium Laureth Sulfate) | 32,0% | 32,0% | 32,0% | 32,0% | 32,0% | 32,0% |
| NaCl | 3,0% | 3,0% | 3,0% | 3,0% | 3,0% | 3,0% |
| Wasser, demineralisiert | 60,0% | 60,0% | 60,0% | 60,0% | 57,5% | 57,5% |
| Sulfosuccinat Beispiel 1, 60%-ig (erfindungsgemäß) | 5,0% | | | | | |
| Sulfosuccinat Beispiel 3, 60%-ig (erfindungsgemäß) | | 5,0% | | | | |
| Sulfosuccinat Beispiel 6, 60%-ig (erfindungsgemäß) | | | 5,0% | | | |
| Sulfomethylsuccinat Beispiel 11, 60%-ig (erfindungsgemäß) | | | | 5,0% | | |
| Sulfosuccinat Beispiel 8, 40%-ig (nicht erfindungsgemäß) | | | | | 7,5% | |
| Vergleichsbeispiel 10, 40%-ig (nicht erfindungsgemäß) | | | | | | 7,5% |

Die sensorischen Testergebnisse sind in Tabelle 2 zusammengefasst.

Tab. 2: Ergebnisse des Handwaschtests I:

| Testformulierung | 1a | 3a | 6a | 11a | V8a | V10a |
|---|---|---|---|---|---|---|
| Anschäumverhalten | 3,2 | 3,4 | 3,3 | 3,2 | 3,1 | 3,0 |
| Schaumvolumen | 2,9 | 3,1 | 3,2 | 2,9 | 2,9 | 2,9 |
| Schaumcremigkeit | 2,9 | 2,9 | 2,9 | 2,9 | 2,8 | 2,9 |
| Hautgefühl während des Waschens | 3,3 | 3,3 | 3,2 | 3,2 | 3,0 | 3,2 |
| Hautglätte | 2,6 | 2,4 | 2,7 | 2,7 | 2,3 | 2,2 |
| Hautweichheit | 2,7 | 2,9 | 2,4 | 2,7 | 2,4 | 2,5 |
| Hautglätte nach 3 min. | 3,5 | 3,4 | 3,4 | 3,5 | 3,2 | 3,3 |
| Hautweichheit nach 3 min. | 3,4 | 3,3 | 3,3 | 3,4 | 3,1 | 3,2 |

In Tabelle 2 sind die Ergebnisse des Handwaschtests I dargestellt. Anhand der Messergebnisse wird ersichtlich, dass die erfindungsgemäßen Formulierung 1a, 2a, 3a und 11a unter Verwendung der erfindungsgemäßen Sulfosuccinate 1, 3 und 6 sowie des Sulfomethylsuccinats 11 überraschenderweise in fast allen Applikationseigenschaften im Vergleich zur Vergleichsformulierung V10a nach dem Stand der Technik und auch im Vergleich zur Formulierung mit Sulfosuccinat Beispiel 8 überlegen oder zumindest gleichwertig waren.

Vor diesem Hintergrund sind die Ergebnisse der erfindungsgemäßen Formulierungen 1a, 3a, 6a und 11a als sehr gut zu bezeichnen und zeigen eine deutliche Verbesserung gegenüber dem Stand der Technik.

Anhand der Messwerte ist ersichtlich, dass das erfindungsgemäße Sulfosuccinat 1 in der Formulierung 1a und das erfindungsgemäße Sulfomethylsuccinat 11 in Formulierung 11a zu einer signifikanten Verbesserung speziell bei der Hautglätte direkt nach dem Waschen führte. Ferner kann man den Messwerten entnehmen, dass das erfindungsgemäße Sulfosuccinat 3 in der Formulierung 3a signifikant bessere Werte beim Anschäumverhalten und der Hautweichheit direkt

nach dem Waschen aufgewiesen hat. Das erfindungsgemäße Sulfosuccinat 6 in der Formulierung 6a zeigte eine deutliche Verbesserung beim Anschäumverhalten, Schaumvolumen und bei der Hautglätte nach dem Waschen.

Neben der Standard-Testformulierung aus Tabelle 1 wurde zudem eine Formulierung mit Zusätzen von nur polyglycoletherfreien Cotensiden untersucht. Es wurde eine Formulierung mit 4,8% aktiv Sodium Cocoamphoacetate, 4,9% aktiv Cocamidopropyl Betaine und 3,6% aktiv Sulfosuccinat getestet (Tabelle 3).

Tab. 3: Testformulierungen für Handwaschtest II:

| Formulierungsbeispiele | 1b | 3b | 6b | V9b | V10b |
|---|---|---|---|---|---|
| TEGO® Betain F 50, Evonik Industries AG, 38%-ig, (INCI: Cocamidopropyl Betaine) | 12,9% | 12,9% | 12,9% | 12,9% | 12,9% |
| REWOTERIC® AM C, Evonik Industries AG, 32%-ig, (INCI: Sodium Cocoamphoacetate) | 15,0% | 15,0% | 15,0% | 15,0% | 15,0% |
| NaCl | 3,0% | 3,0% | 3,0% | 3,0% | 3,0% |
| Wasser, demineralisiert | 63,1% | 63,1% | 63,1% | 60,1% | 60,1% |
| Sulfosuccinat Beispiel 1, 60%-ig (erfindungsgemäß) | 6,0% | | | | |
| Sulfosuccinat Beispiel 3, 60%-ig (erfindungsgemäß) | | 6,0% | | | |
| Sulfosuccinat Beispiel 6, 60%-ig (erfindungsgemäß) | | | 6,0% | | |
| Sulfosuccinat Beispiel 9, 40%-ig (nicht erfindungsgemäß) | | | | 9,0% | |
| Vergleichsbeispiel 10, 40%-ig (nicht erfindungsgemäß) | | | | | 9,0% |

Die sensorischen Testergebnisse sind in Tabelle 4 zusammengefasst.

Tab. 4: Ergebnisse des Handwaschtests II:

| Testformulierung | 1b | 3b | 6b | V9b | V10b |
|---|---|---|---|---|---|
| Anschäumverhalten | 3,4 | 3,2 | 3,4 | 3,2 | 3,2 |
| Schaumvolumen | 3,6 | 3,4 | 3,4 | 3,4 | 3,4 |
| Schaumcremigkeit | 3,6 | 3,7 | 3,4 | 3,1 | 3,3 |
| Hautgefühl während des Waschens | 3,8 | 4,0 | 3,8 | 3,7 | 3,8 |
| Hautglätte | 2,6 | 2,5 | 2,6 | 2,5 | 2,4 |
| Hautweichheit | 2,8 | 2,8 | 2,8 | 3,0 | 2,9 |
| Hautglätte nach 3 min. | 3,6 | 3,4 | 3,6 | 3,4 | 3,3 |
| Hautweichheit nach 3 min. | 3,3 | 3,2 | 3,3 | 3,2 | 3,2 |

In Tabelle 4 sind die Ergebnisse des Handwaschtests II dargestellt. Anhand der Messergebnisse wird ersichtlich, dass die erfindungsgemäßen Formulierung 1b, 3b und 6b unter Verwendung der erfindungsgemäßen Sulfosuccinate 1, 3 und 6 überraschenderweise in fast allen Applikationseigenschaften (außer bei der Hautweichheit direkt nach dem Waschen) im Vergleich zur Vergleichsformulierung V10b nach dem Stand der Technik und auch im Vergleich zur Formulierung V9b mit Sulfosuccinat Beispiel 9 überlegen oder zumindest gleichwertig waren.

Anhand der Messwerte ist ersichtlich, dass das erfindungsgemäße Sulfosuccinat 1 in der Formulierung 1b zu einer signifikanten Verbesserung speziell bei der Schaumcremigkeit und der Hautglätte 3 min nach dem Waschen geführt hat. Ferner kann man den Messwerten entnehmen, dass das erfindungsgemäße Sulfosuccinat 3 in der Formulierung 3b signifikant bessere Werte bei der Schaumcremigkeit aufweist. Das erfindungsgemäße Sulfosuccinat 6 in der Formulierung 6b zeigte eine deutliche Verbesserung bei der Hautglätte nach 3 min.

Vor diesem Hintergrund sind die Ergebnisse der erfindungsgemäßen Formulierungen 1b, 3b und 6b als sehr gut zu bezeichnen und zeigen eine Verbesserung gegenüber dem Stand der Technik.

*Austestung der Verdickung von tensidischen Formulierungen:*

Zur Bewertung der Verdickungseignung von Formulierungen mit den erfindungsgemäßen Sulfosuccinaten wurden mehrere unterschiedliche Tensidlösungen angesetzt, mit unterschiedlichen Verdickern versetzt und die erreichten Viskositäten der Formulierungen gemessen.

Tab. 5: Testformulierungen für Viskositätsmessungen I:

| Formulierungsbeispiele | 1c | 3c | 6c | V10c |
|---|---|---|---|---|
| Texapon® NSO, BASF Cognis, 28%-ig, (INCI: Sodium Laureth Sulfate) | 32% | 32% | 32% | 32,0% |
| NaCl | 5% | 5% | 5% | 5,0% |
| Wasser, demineralisiert | 58% | 58% | 58% | 55,5% |
| Sulfosuccinat Beispiel 1, 60%-ig (erfindungsgemäß) | 5% | | | |
| Sulfosuccinat Beispiel 3, 60%-ig (erfindungsgemäß) | | 5% | | |
| Sulfosuccinat Beispiel 6, 60%-ig (erfindungsgemäß) | | | 5% | |
| Vergleichsbeispiel 10, 40%-ig (nicht erfindungsgemäß) | | | | 7,5% |
| Viskosität (mPa·s) | 3168 | 3072 | 4224 | 2340 |

Tab. 6: Testformulierungen für Viskositätsmessungen II:

| Formulierungsbeispiele | 1d | 3d | 6d | V10d |
|---|---|---|---|---|
| Texapon® NSO, BASF Cognis, 28%-ig, (INCI: Sodium Laureth Sulfate) | 32% | 32% | 32% | 32,0% |
| ANTIL® 120 Plus, Evonik Industries AG, (INCI: PEG-120 Methyl Glucose Dioleate) | 3% | 3% | 3% | 3,0% |
| Wasser, demineralisiert | 60% | 60% | 60% | 57,5% |
| Sulfosuccinat Beispiel 1, 60%-ig (erfindungsgemäß) | 5% | | | |
| Sulfosuccinat Beispiel 3, 60%-ig (erfindungsgemäß) | | 5% | | |
| Sulfosuccinat Beispiel 6, 60%-ig (erfindungsgemäß) | | | 5% | |
| Vergleichsbeispiel 10, 40%-ig (nicht erfindungsgemäß) | | | | 7,5% |
| Viskosität (mPa·s) | 6168 | 6072 | 7224 | 5870 |

Tab. 7: Testformulierungen für Viskositätsmessungen III:

| Formulierungsbeispiele | 1e | 3e | 6e | 11e | V10e |
|---|---|---|---|---|---|
| Texapon® NSO, BASF Cognis, 28%-ig, (INCI: Sodium Laureth Sulfate) | 17,9% | 17,9% | 17,9% | 17,9% | 17,9% |
| TEGO® Betain F 50, Evonik Industries AG, 38%-ig, (INCI: Cocamidopropyl Betaine) | 6,6% | 6,6% | 6,6% | 6,6% | 6,6% |
| ANTIL® 120 Plus, Evonik Industries AG, (INCI: PEG-120 Methyl Glucose Dioleate) | 0,7% | 0,7% | 0,7% | 0,7% | 0,7% |
| NaCl | 1,0% | 1,0% | 1,0% | 1,0% | 1,0% |
| Wasser, demineralisiert | 69,6% | 69,6% | 69,6% | 69,6% | 67,5% |
| Sulfosuccinat Beispiel 1, 60%-ig (erfindungsgemäß) | 4,2% | | | | |
| Sulfosuccinat Beispiel 3, 60%-ig (erfindungsgemäß) | | 4,2% | | | |
| Sulfosuccinat Beispiel 6, 60%-ig (erfindungsgemäß) | | | 4,2% | | |

(fortgesetzt)

| Formulierungsbeispiele | 1e | 3e | 6e | 11e | V10e |
|---|---|---|---|---|---|
| Sulfomethylsuccinat Beispiel 11, 60%-ig (erfindungsgemäß) | | | | 4,2% | |
| Vergleichsbeispiel 10, 40%-ig (nicht erfindungsgemäß) | | | | | 6,3% |
| Viskosität (mPa·s) | 4200 | 4106 | 4650 | 4170 | 1780 |

Tab. 8: Testformulierungen für Viskositätsmessungen IV:

| Formulierungsbeispiele | 1f | 3f | 6f | V8f | V9f | V10f |
|---|---|---|---|---|---|---|
| Texapon® NSO, BASF Cognis, 28%-ig, (INCI: Sodium Laureth Sulfate) | 17,9% | 17,9% | 17,9% | 17,9% | 17,9% | 17,9% |
| TEGO® Betain F 50, Evonik Industries AG, 38%-ig, (INCI: Cocamidopropyl Betaine) | 6,6% | 6,6% | 6,6% | 6,6% | 6,6% | 6,6% |
| REWOMID® DC 212 S, Evonik Industries AG, (INCI: Cocamide DEA) | 3,8% | 3,8% | 3,8% | 3,8% | 3,8% | 3,8% |
| NaCl | 1,0% | 1,0% | 1,0% | 1,0% | 1,0% | 1,0% |
| Wasser, demineralisiert | 66,5% | 66,5% | 66,5% | 64,4% | 64,4% | 64,4% |
| Sulfosuccinat Beispiel 1, 60%-ig (erfindungsgemäß) | 4,2% | | | | | |
| Sulfosuccinat Beispiel 3, 60%-ig (erfindungsgemäß) | | 4,2% | | | | |
| Sulfosuccinat Beispiel 6, 60%-ig (erfindungsgemäß) | | | 4,2% | | | |
| Sulfosuccinat Beispiel 8, 40%-ig (nicht erfindungsgemäß) | | | | 6,3% | | |
| Sulfosuccinat Beispiel 9, 40%-ig (nicht erfindungsgemäß) | | | | | 6,3% | |
| Vergleichsbeispiel 10, 40%-ig (nicht erfindungsgemäß) | | | | | | 6,3% |
| Viskosität (mPa·s) | 4469 | 3947 | 6005 | 2445 | 2420 | 2320 |

Tab. 9: Testformulierungen für Viskositätsmessungen V:

| Formulierungsbeispiele | 1g | 3g | 6g | V8g | V9g | V10g |
|---|---|---|---|---|---|---|
| Texapon® LS 35, BASF Cognis, 30%-ig, (INCI: Sodium Lauryl Sulfate) | 6,7% | 6,7% | 6,7% | 6,7% | 6,7% | 6,7% |
| TEGO® Betain F 50, Evonik Industries AG, 38%-ig, (INCI: Cocamidopropyl Betaine) | 10,3% | 10,3% | 10,3% | 10,3% | 10,3% | 10,3% |
| REWOTERIC® AM C, Evonik Industries AG, 32%-ig, (INCI: Sodium Cocoamphoacetate) | 11,9% | 11,9% | 11,9% | 11,9% | 11,9% | 11,9% |
| ANTIL® SPA 80, Evonik Industries AG, (INCI: Isostearamide MIPA; Glyceryl Laurate) | 0,5% | 0,5% | 0,5% | 0,5% | 0,5% | 0,5% |
| Wasser, demineralisiert | 67,3% | 67,3% | 67,3% | 65,6% | 65,6% | 65,6% |
| Sulfosuccinat Beispiel 1, 60%-ig (erfindungsgemäß) | 3,3% | | | | | |
| Sulfosuccinat Beispiel 3, 60%-ig (erfindungsgemäß) | | 3,3% | | | | |
| Sulfosuccinat Beispiel 6, 60%-ig (erfindungsgemäß) | | | 3,3% | | | |
| Sulfosuccinat Beispiel 8, 40%-ig (nicht erfindungsgemäß) | | | | 5,0% | | |

(fortgesetzt)

| Formulierungsbeispiele | 1g | 3g | 6g | V8g | V9g | V10g |
|---|---|---|---|---|---|---|
| Sulfosuccinat Beispiel 9, 40%-ig (nicht erfindungsgemäß) | | | | | 5,0% | |
| Vergleichsbeispiel 10, 40%-ig (nicht erfindungsgemäß) | | | | | | 5,0% |
| Viskosität (mPa·s) | 4320 | 4619 | 4363 | 810 | 452 | 2880 |

In den Tabellen 5 - 9 wird gezeigt, dass die erfindungsgemäßen Formulierungen mit den erfindungsgemäßen Sulfosuccinaten 1, 3, 6 und auch dem Sulfomethylsuccinat 11 im Vergleich zu den Vergleichsformulierungen V8 - V10 deutlich höhere Viskositäten in speziellen Formulierungen mit unterschiedlichsten Verdickern geliefert haben.
Tabellen 5 und 6 zeigen ein Standardtensidsystem mit 9% aktiv Sodium Laureth Sulfate und 3% aktiv Sulfosuccinat. Sowohl mit Natriumchlorid als auch mit dem polymeren, hydrophilen Verdicker PEG-120 Methyl Glucose Dioleate ließen sich die erfindungsgemäßen Formulierungen deutlich besser verdicken als die Vergleichsformulierung V10a.
In den Formulierungen der Tabellen 7 und 8 wurde ein sehr mildes und niedrig konzentriertes Tensidsystem verwendet. Die Gesamtmenge an waschaktiver Substanz betrug nur 10% (5% aktiv Sodium Laureth Sulfate, 2,5% aktiv Cocamidopropyl Betaine und 2,5% aktiv Sulfosuccinat). Auch diese Formulierungen ließen sich bei Zusatz der erfindungsgemäßen Sulfosuccinate deutlich besser verdicken als die entsprechenden Vergleichsformulierungen mit Vergleichsbeispiel 10. Der Effekt zeigte sich sowohl bei Einsatz des polymeren, hydrophilen Verdickers PEG-120 Methyl Glucose Dioleate als auch mit dem hydrophoben Verdicker Cocamide DEA. In Tabelle 8 wird zudem deutlich, dass hier die erfindungsgemäßen Sulfosuccinate 1, 3 und 6 wesentlich höhere Viskositäten erreicht haben als die Vergleichsbeispiele Sulfosuccinat Beispiel 8 und 9 nach dem Stand der Technik.
Auch im sehr milden, polyglycoletherfreien Tensidsystem der Tabelle 9 (2% aktiv Sodium Lauryl Sulfate, 3,8% Sodium Cocoamphoacetate, 3,9% aktiv Cocamidopropylbetaine und 2% aktiv Sulfosuccinat) ließen sich mit 0.5% des hydrophoben Verdickers ANTIL® SPA 80 (INCI: Isostearamide MIPA; Glyceryl Laurate) deutlich höhere Viskositäten erzielen als mit dem Vergleichsbeispiel 10 nach dem Stand der Technik. Auch die Vergleichsbeispiele Sulfosuccinat Beispiel 8 und 9 nach dem Stand der Technik fielen in diesem System überraschenderweise extrem stark ab in der Viskosität.

*Weitere Formulierungsbeispiele Personal Care:*

Die in den nachfolgenden Tabellen angegebenen Formulierungsbeispiele zeigen exemplarische Vertreter einer Vielzahl von möglichen erfindungsgemäßen Zusammensetzungen.

Falls die Herstellung der Formulierung zuvor die getrennte Zubereitung bzw. Mischung von Formulierungsbestandteilen erfordert, wird dieses als mehrphasige Zubereitung bezeichnet. Falls eine zweiphasige Herstellung erforderlich ist, werden die beiden Phasen mit A und B in den angegebenen Tabellen gekennzeichnet. Bei dreiphasigen Prozessen werden die drei Phasen mit A, B und C benannt.
Wenn nicht anders angegeben, handelt es sich bei den Angaben in den folgenden Tabellen um Angaben in Gew.-% Aktivstoff. Wird ein Aktivgehalt angegeben, ist die Prozentangabe die eingesetzte Menge an diesem nicht 100%-igem Gemisch und nicht die Aktivstoffmenge.

Tab. 10: Mildes, sulfat-freies Haar- und Körperreinigungsmittel

| | |
|---|---|
| Cocamidopropyl Betaine | 4,9% |
| Sodium Cocoamphoacetate | 4,5% |
| Sulfosuccinat Beispiel 1, 60%-ig | 6,0% |
| Sucrose Cocoate | 1,5% |
| PEG-120 Methyl Glucose Dioleate | 2,0% |
| Polyquaternium-10 | 0,2% |
| Water | ad 100,0% |
| Parfum, Preservative | q.s. |
| Citric Acid, 30% | ad pH 5,5 |

Tab. 11: Mildes, sulfat-freies Körperreinigungsmittel

| | |
|---|---|
| Sodium Cocoamphoacetate | 4,8% |
| Sulfosuccinat Beispiel 2, 60%-ig | 6,8% |
| Sorbitan Sesquicaprylate | 0,9% |
| Water | ad 100,0% |
| Cocamidopropyl Betaine | 4,2% |
| Citric Acid, 30% | ad pH 5,0 |
| PEG-120 Methyl Glucose Dioleate | 1,4% |
| Parfum, Preservative | q.s. |

Tab. 12: sulfat-freies Baby Shampoo

| | |
|---|---|
| Sodium Cocoamphoacetate | 3,2% |
| Isostearamide MIPA; Glyceryl Laurate | 1,0% |
| Water | ad 100,0% |
| Sulfosuccinat Beispiel 6, 60%-ig | 10,0% |
| Palmitamidopropyltrimonium Chloride | 1,0% |
| PEG-200 Hydrogenated Glyceryl Palmate; PEG-7 Glyceryl Cocoate | 2,6% |
| Citric Acid, 30% | ad pH 5,7 |
| Parfum, Preservative | q.s. |

Tab. 13: Mildes, PEG- und sulfat-freies Haar- und Körperreinigungsmittel

| | |
|---|---|
| Lauryl Glucoside | 4,4% |
| Cocamidopropyl Betaine | 5,7% |
| Sodium Cocoamphoacetate | 3,6% |
| Sulfosuccinat Beispiel 2, 60%-ig | 2,9% |
| Hydroxypropyl Methylcellulose | 0,5% |
| Isostearamide MIPA; Glyceryl Laurate | 1,4% |
| Polyquaternium-7 | 0,5% |
| Water | ad 100,0% |
| Parfum, Preservative | q.s. |
| Citric Acid, 30% | ad pH 5,5 |

Tab. 14: Duschgel

| | |
|---|---|
| Sodium Laureth Sulfate | 4,0% |
| Sulfosuccinat Beispiel 11, 60%-ig | 3,0% |
| Sodium Cocoyl Hydrolyzed Wheat Protein Glutamate | 1,5% |
| Water | ad 100,0% |
| Cocamidopropyl Betaine | 3,0% |

(fortgesetzt)

| | |
|---|---|
| Capryl/Capramidopropyl Betaine | 1,0% |
| Polyquaternium-7 | 0,5% |
| PEG-200 Hydrogenated Glyceryl Palmate (and) PEG-7 Glyceryl Cocoate | 1,8% |
| Parfum | q.s. |
| Preservative | q.s. |

Tab. 15: Shampoo

| | |
|---|---|
| Water | ad 100,0% |
| Cocamidopropyl Betaine | 4,0% |
| Sodium Lauryl Sulfoacetate | 3,0% |
| Sulfosuccinat Beispiel 2, 60%-ig | 3,0% |
| Sodium Lauroyl Sarcosinate | 1,0% |
| Glycol Distearate | 0,9% |
| Sodium Chloride | 0,9% |
| Decyl Glucoside | 0,5% |
| Polyquaternium-10 | 0,3% |
| PPG-5-Ceteth-20 | 0,5% |
| Coco-Betaine | 0,5% |
| PEG-55 Propylene Glycol Oleate | 0,4% |
| Propylene Glycol | 0,3% |
| Salicylic Acid | 0,2% |
| Carbomer | 0,3% |
| Sodium Hydroxide | 0,2% |
| Citric Acid | ad pH 5,5 |
| Preservative, Parfum, Dyes | q.s. |

Tab. 16: Körperreinigungsmittel, PEG- & Sulfat-frei

| | |
|---|---|
| Water | ad 100,0% |
| Sodium Cocoamphoacetate | 5,0% |
| Sulfosuccinat Beispiel 6, 60%-ig | 2,0% |
| Cocamidopropyl Betaine | 3,0% |
| Cocamidopropyl Betaine (and) Glyceryl Laurate | 1,0% |
| Citric Acid, 30% | ad pH 5,0 |
| Preservative, Parfum | q.s. |

Tab. 17: Wasch-Emulsion für empfindliche und problematische Haut

| | |
|---|---|
| Water | ad 100,0% |
| Sodium C14-16 Olefin Sulfonate | 4,0% |

(fortgesetzt)

| | |
|---|---|
| Sodium Laureth Sulfate | 3,0% |
| Sulfosuccinat Beispiel 3, 60%-ig | 4,0% |
| Sodium Chloride | 2,0% |
| Laureth-2 | 1,0% |
| Panthenol | 0,5% |
| Glycol Distearate | 0,5% |
| Saccaride Isomerate | 0,2% |
| Allantoin | 0,2% |
| Niacinamide | 0,1% |
| Pyridoxine HCl | 0,1% |
| Glycine | 0,1% |
| Alanine | 0,1% |
| Lysine | 0,1% |
| Biotin | 0,1% |
| Glycerin | 0,5% |
| Sodium Lauroyl Glutamate | 0,5% |
| Sodium Citrate | 0,5% |
| Cocamidopropyl Betaine | 0,5% |
| Sorbitan Laurate | 0,4% |
| PEG-120 Methyl Glucose Dioleate | 0,3% |
| Preservative, Parfum | q.s. |

Tab. 18: Kids Dusche & Shampoo

| | |
|---|---|
| Water | ad 100% |
| Sulfosuccinat Beispiel 3, 60%-ig | 6,0% |
| Cocamidopropyl Hydroxysultaine | 2,5% |
| Cocamidopropyl Betaine | 1,5% |
| Caprylyl/Capryl Glucoside | 1,0% |
| PEG-18 Glyceryl Oleate/Cocoate | 0,7% |
| Laureth-2 | 0,5% |
| Sodium Chloride | 0,5% |
| Panthenol | 0,2% |
| Niacinamide | 0,1% |
| Pyridoxan Hydrochloride | 0,1% |
| Polyquaternium-10 | 0,3% |
| Glycerin | 0,3% |
| Inulin | 0,1% |
| Citric Acid | ad pH 5,7 |

(fortgesetzt)

| | |
|---|---|
| Preservative, Parfum, Dyes | q.s. |

Tab. 19: Mildes Baby Shampoo

| | |
|---|---|
| Water | ad 100% |
| Sulfosuccinat Beispiel 11, 60%-ig | 5,5% |
| Isostearamide MIPA (and) Glyceryl Laurate | 1,7% |
| PEG-7 Glyceryl Cocoate | 0,5% |
| Sodium Cocoamphoacetate | 3,0% |
| Palmitamidopropyltrimonium Chloride | 2,3% |
| Citric Acid, 30% | ad pH 6,0 |
| Preservative, Parfum, Dyes | q.s. |

Tab. 20: Milder Gesichtsreinigungsschaum

| | |
|---|---|
| Water | ad 100% |
| Sulfosuccinat Beispiel 5, 60%-ig | 8,5% |
| Capryl/Capramidopropyl Betaine | 3,0% |
| Polyglyceryl-3 Caprate | 0,5% |
| Creatine | 0,2% |
| Hydroxypropyl Methylcellulose | 0,5% |
| Sodium Lactate (and) Sodium PCA (and) Glycine (and) Fructose (and) Urea (and) Niacinamide (and) Inositol (and) Sodium Benzoate (and) Lactic Acid | 1,0% |
| Preservative, Parfum | q.s. |

Tab. 21 a) und b): Körperreinigungsmittel

| | | |
|---|---|---|
| Water | ad 100% | ad 100% |
| Sodium Lauryl Sulfate | 9% | 9% |
| Sulfosuccinat Beispiel 1, 60%-ig | 5% | 5% |
| PEG-120 Methyl Glucose Dioleate | 3% | |
| Sodium Chloride | | 5% |
| Preservative, Parfum | q.s. | q.s. |

Tab. 22 a) und b): Körperreinigungsmittel

| | | |
|---|---|---|
| Water | ad 100% | ad 100% |
| Sodium Lauryl Sulfate | 5,0% | 5,0% |
| Cocamidopropyl Betaine | 2,5% | 2,5% |
| Sulfosuccinat Beispiel 3, 60%-ig | 4,2% | 4,2% |
| PEG-120 Methyl Glucose Dioleate | 0,7% | |

(fortgesetzt)

| | | |
|---|---|---|
| Cocamide DEA | | 3,8% |
| Sodium Chloride | 1,0% | 1,0% |
| Preservative, Parfum | q.s. | q.s. |

Tab. 23 a) und b): Milde, PEG-freie Körperreinigungsmittel

| | | |
|---|---|---|
| Water | ad 100% | ad 100% |
| Cocamidopropyl Betaine | 3,9% | 3,9% |
| Sodium Cocoamphoacetate | 3,8% | 3,8% |
| Sodium Lauryl Sulfate | 2,0% | 2,0% |
| Sulfosuccinat Beispiel 1, 60%-ig | 3,3% | 3,3% |
| Sorbitan Sesquicaprylate | 0,5% | |
| Isostearamide MIPA; Glyceryl Laurate | | 0,5% |
| Citric Acid | ad pH 5,0 | ad pH 5,0 |
| Preservative, Parfum | q.s. | q.s. |

Tab. 24: Mildes, PEG- und sulfat-freies Körperreinigungsmittel

| | |
|---|---|
| Water | ad 100% |
| Cocamidopropyl Betaine | 5,7% |
| Lauryl Glucoside | 4,4% |
| Sodium Cocoamphoacetate | 3,6% |
| Sulfosuccinat Beispiel 3, 60%-ig | 2,8% |
| Isostearamide MIPA; Glyceryl Laurate | 1,4% |
| Citric Acid | ad pH 5,0 |
| Preservative, Parfum | q.s. |

Tab. 25: Mildes Gesichtsreinigungsgel; PEG- und sulfat-frei

| | |
|---|---|
| Water | ad 100% |
| Coco Glucoside | 4,0% |
| Sodium Cocoyl Glutamate, Disodium Cocoyl Glutamate | 4,0% |
| Glycerin | 3,0% |
| Xanthan Gum | 1,5% |
| Sulfosuccinat Beispiel 6, 60%-ig | 1,7% |
| Preservative, Parfum | q.s. |

Tab. 26: Mildes Gesichtsreinigungsgel; PEG- und sulfat-frei, Ecocert-conform

| | |
|---|---|
| Water | ad 100% |
| Sodium Cocoamphoacetate | 3,2% |

(fortgesetzt)

| | |
|---|---|
| Polyglyceryl-3 Caprate | 3,0% |
| Cocamidopropyl Betaine | 2,3% |
| Capryl/Capramidopropyl Betaine | 2,3% |
| Xanthan Gum | 1,5% |
| Sodium Chloride | 1,5% |
| Coamidopropyl Betaine; Glyceryl Laurate | 1,3% |
| Sulfosuccinat Beispiel 1, 60%-ig | 1,7% |
| Sucrose Cocoate | 0,6% |
| Citric Acid | ad pH 5,0 |
| Preservative, Parfum | q.s. |

Tab. 27: Weitere Formulierungsbeispiele

| | a | b | c | d | e | f | g | h | i | j |
|---|---|---|---|---|---|---|---|---|---|---|
| Water | ad 100% | | | | | | | | | |
| Sulfosuccinat Beispiel 1, 60%-ig | 15,0% | 7,0% | 4,0% | 4,5% | 7,0% | 5,5% | 8,0% | 4,0% | 4,5% | 3,0% |
| Disodium Ricinoleamido MEA-Sulfosuccinate | - | 4,0% | 5,0% | - | - | 1,0% | - | | | |
| Sodium Laureth Sulfate | - | - | - | 7,0% | - | - | - | 4,5% | - | - |
| Ammonium Lauryl Sulfate | - | - | - | - | 5,0% | - | - | - | 3,5% | - |
| Cocamidopropyl Betaine | - | - | - | - | - | 6,0% | - | 2,5% | - | 4,5% |
| Sodium Cocoamphoacetate | - | - | 5,0% | - | - | - | - | - | 3,5% | - |
| Coco Glucoside | - | - | - | - | - | - | 5,5% | - | - | 2,5% |
| Sorbitol | - | 1,0% | 0,5% | - | - | - | 1,0% | - | - | 0,5% |
| Sucrose Cocoate | 0,5% | - | 1,0% | 1,0% | 1,0% | 0,5% | 0,3% | 1,0% | 1,0% | - |
| Hydroxypropyl Methylcellulose | 0,5% | 0,5% | 0,3% | - | - | 0,3% | 0,5% | - | - | 0,1% |
| Polyglyceryl-4 Caprate | - | - | - | - | - | - | - | 0,5% | - | - |
| Hydroxypropyl Guar Hydroxypropyltrimonium Chloride | 0,2% | 0,2% | 0,2% | 0,3% | 0,2% | 0,2% | 0,2% | 0,2% | 0,2% | 0,2% |
| Dimethicone (and) Dimethiconol | - | - | - | - | - | - | - | 0,3% | - | - |
| Silicone Quaternium-22 | - | - | - | 1,0% | 0,5% | - | - | - | - | - |
| Amodimethicone | - | - | 0,1% | - | - | - | 0,5% | - | - | - |
| PEG-3 Distearate | - | - | 0,5% | - | 0,5% | - | - | 0,5% | - | 0,5% |
| Acrylates / C10-30 Alkyl Acrylate Crosspolymer | 1,0% | - | - | - | 0,5% | - | - | - | 0,5% | - |
| Sodium Hydroxide, 25% | 1,5% | - | - | - | 0,8% | - | - | - | 0,7% | - |
| Cocamide MEA | - | 1,5% | - | - | - | - | - | - | 1,0% | 0,5% |
| Sorbitan Sesquicaprylate | - | 0,5% | - | 0,5% | - | 1,2% | 1,0% | - | - | 1,3% |
| Sodium Chloride | 1,0% | 1,0% | - | 0,5% | 0,3% | 1,5% | - | 2,0% | 1,5% | - |
| PEG-200 Hydrogenated Glyceryl Palmate; PEG-7 Glyceryl Cocoate | - | - | - | 0,4% | - | - | - | 0,6% | - | - |
| Xanthan Gum | - | 0,5% | 1,2% | - | - | - | 1,0% | - | - | 0,5% |
| Zinc Pyrithione | - | - | - | 0,1% | - | - | - | 0,1% | - | - |

(fortgesetzt)

| | a | b | c | d | e | f | g | h | i | j |
|---|---|---|---|---|---|---|---|---|---|---|
| Pentylene Glycol | - | - | - | 0,1% | - | - | 0,4% | - | - | - |
| Panthenol | 0,1% | 0,1% | 0,1% | 0,1% | 0,1% | 0,1% | 0,1% | 0,1% | 0,1% | 0,1% |
| Citric Acid | ad pH 5,5 | | | | | | | | | |
| Preservative, Parfum, Dyes | q.s. | | | | | | | | | |

Tab. 28: Weitere Formulierungsbeispiele

| | a | b | c | d | e | f | g | h | i | j |
|---|---|---|---|---|---|---|---|---|---|---|
| Water | ad 100% | | | | | | | | | |
| Sulfosuccinat Beispiel 3, 60%-ig | 4,0% | 3,0% | 4,5% | 4,5% | 7,0% | 5,5% | 3,0% | 4,0% | 1,5% | 3,0% |
| Sodium Cocoyl Glycinate | 4,0% | - | - | - | - | - | 2,0% | 1,0% | - | - |
| Sodium Laureth Sulfate | - | 5,0% | 7,0% | - | - | - | - | 4,0% | 4,5% | - |
| Undecylenamidopropyl Betaine | - | 3,0% | 4,0% | 5,0% | - | - | - | - | - | - |
| Cocamidopropyl Betaine | 4,0% | 2,0% | - | 4,0% | 4,5% | - | - | 1,5% | 1,0% | 5,5% |
| Lauroyl Sarcosine | - | - | - | - | 3,0% | 2,0% | 2,0% | - | 2,5% | 1,0% |
| Sodium Cocoyl Isethionate | - | - | - | - | - | - | 2,0% | 1,0% | - | 2,5% |
| Coco Glucoside | - | - | - | 1,0% | - | 5,5% | 2,5% | - | 1,0% | - |
| Glycerin | 0,3% | - | 0,5% | 0,3% | - | 1,0% | 1,0% | - | 0,5% | 0,2% |
| Hydroxypropyl Methylcellulose | 0,5% | - | 0,3% | 0,2% | - | - | 0,2% | - | - | - |
| Glyceryl Caprylate | 0,2% | - | 0,1% | - | - | 0,3% | - | - | 0,5% | - |
| Polyglyceryl-4 Caprate | - | - | - | 0,5% | - | - | 0,5% | - | - | - |
| PEG-40 Hydrogenated Castor Oil | - | 0,2% | - | - | - | - | - | - | - | - |
| Diethylhexyl Carbonate | - | - | 0,1% | - | - | - | - | - | - | - |
| Caprylic/Capric | 0,1% | - | - | 0,2% | - | - | - | - | 0,1% | - |
| Triglyceride | | | | | | | | | | |
| Dicaprylylether | - | 0,3% | - | - | - | 0,5% | - | - | - | - |
| Polyquaternium-10 | 0,2% | 0,3% | 0,2% | 0,3% | 0,2% | 0,2% | 0,2% | 0,3% | - | 0,2% |
| Bis-PEG/PPG-20/20 Dimethicone | - | 0,1% | - | - | - | - | - | 0,1% | - | - |
| Glycol Distearate | - | 0,5% | 0,3% | 0,5% | - | - | - | 0,3% | - | 0,5% |
| Carbomer | - | - | - | - | 0,5% | - | - | 0,5% | - | - |
| Sodium Hydroxide, 25% | - | - | - | - | 0,8% | - | - | 0,7% | - | - |
| Cocamide MEA | - | - | - | 0,3% | - | - | - | - | 1,0% | 0,5% |
| Sodium Chloride | 1,0% | 1,5% | 0,5% | 0,5% | 0,3% | 0,5% | - | 1,0% | 1,5% | - |
| PEG-120 Methyl Glucose Dioleate | - | 0,3% | 0,8% | - | - | - | - | - | 0,2% | - |
| Xanthan Gum | - | - | - | 0,5% | - | 1,0% | 0,8% | - | - | 0,3% |
| Climbazol | - | 0,1% | - | 0,1% | - | - | - | - | - | - |
| Octopirox | - | - | 0,1% | - | - | - | - | 0,1% | - | - |
| Hydrolyzed Wheat Protein | 0,1% | - | 0,1% | - | - | 0,1% | 0,1% | 0,1% | - | 0,1% |
| Panthenol | 0,1% | 0,1% | 0,1% | 0,1% | 0,1% | 0,1% | 0,1% | - | 0,1% | 0,1% |
| Citric Acid | ad pH 5,5 | | | | | | | | | |
| Preservative, Parfum, Dyes | q.s. | | | | | | | | | |

Tabelle 29: Liste der verwendeten Rohstoffe:

| INCI-Name: | Handelsname: |
|---|---|
| Verdicker/Stabilisatoren: | |
| Carbomer | TEGO Carbomer 140, Evonik Industries AG, 100% |
| Acrylates / C10-30 Alkyl Acrylate Cross polymer | TEGO Carbomer 341 ER, Evonik Industries AG, 100% |
| Cocamide DEA | REWOMID DC 212 S, Evonik Industries AG, 100% |
| Cocamide MEA | REWOMID D 212, Evonik Industries AG, 100% |
| Isostearamide MIPA; Glyceryl Laurate | ANTIL SPA 80, Evonik Industries AG, 100% |
| Sorbitan Sesquicaprylate | ANTIL Soft SC, Evonik Industries AG, 100% |
| Sorbitan Laurate | TEGO SML, Evonik Industries AG, 100% |
| PEG-55 Propylene Glycol Oleate | ANTIL 141 liquid, Evonik Industries AG |
| PEG-120 Methyl Glucose Dioleate | ANTIL 120 Plus, Evonik Industries AG, 100% |
| PEG-18 Glyceryl Oleate/Cocoate | ANTIL 171, Evonik Industries AG, 100% |
| PEG-200 Hydrogenated Glyceryl Palmate; PEG-7 Glyceryl Cocoate | REWODERM LI S 80, Evonik Industries AG, 100% |
| Xanthan Gum | Keltrol CG-SFT, CP Kelco, 100% |
| Perlglanzmittel: | |
| PEG-3 Distearate | Cutina TS, BASF Cognis, 100% |
| Glycol Distearate | TEGIN G 1100, Evonik Industries AG, 100% |
| Silicon Additive: | |
| Amodimethicone | DC 949, Dow Corning, 100% |
| Dimethicone (and) Dimethiconol | DC 1503. Dow Corning |
| Silicone Quaternium-22 | ABIL T Quat 60, Evonik Industries AG, 65% |
| Bis-PEG/PPG-20/20 Dimethicone | ABIL B 8832, Evonik Industries AG, 100% |
| Pflegepolymere/Filmformer: | |
| Polyquaternium-7 | Merquat 550, Nalco, 100% |
| Polyquaternium-10 | Polymer JR 400, Amerchol, 100% |
| Hydroxypropyl Guar Hydroxypropyltrimonium Chloride | Jaguar C-162, Rhodia, 100% |
| Wirkstoffe: | |
| Panthenol | D-Panthenol USP, BASF, 100% |
| Allantoin | Allantoin, DSM |
| Niacinamide | Niacinamide, USP, DSM Nutrional Products, 100% |

(fortgesetzt)

| INCI-Name: | Handelsname: |
|---|---|
| Creatine | TEGO Cosmo C 100, Evonik Industries AG, 100% |
| Sodium Lactate; Sodium PCA; Glycine; Fructose; Urea; Niacinamide; Inositol; Sodium Benzoate; Lactic Acid | LACTIL, Evonik Industries AG, 100% |
| Salicylic Acid | AEC Salicylic Acid, A & E Connock |
| Pyridoxine HCl | Vitamin B6 Hydrochloride, DSM |
| Hydrolyzed Wheat Protein | Gluadin WLM, BASF Cognis |
| Anti-Schuppen-Wirkstoffe: | |
| Climbazol | Crinipan AD, Haarmann & Reimer Fragrance GmbH, 100% |
| Octopirox | Octopirox, Clariant |
| Zinc Pyrithione | Zinc-Pyrion NF, WeylChem, 48% |
| Konditioner: | |
| Palmitamidopropyltrimonium Chloride | VARISOFT PATC, Evonik Industries AG, 60% |
| Tenside: | |
| Cocamidopropyl Betaine | TEGO Betain F 50, Evonik Industries AG, 38% |
| Capryl/Capramidopropyl Betaine | TEGO Betain 810, Evonik Industries AG, 35% |
| Undecylenamidopropyl Betaine | REWOTERIC AM B U 185, Evonik Industries AG, 30% |
| Coco-Betaine | Dehyton AB 30, BASF Cognis, 31% |
| Sodium Cocoamphoacetate | REWOTERIC AM C, Evonik Industries AG, 32% |
| Disodium Ricinoleamido MEA-Sulfosuccinate | REWODERM S 1333, Evonik Industries AG, 40% |
| Sodium Lauryl Sulfoacetate | Lathanol LAL, Stepan |
| Sodium Laureth Sulfate | Texapon NSO, BASF Cognis, 28% bzw TEXAPON N 70, 70% |
| Sodium Lauryl Sulfate | Texapon LS 35, BASF Cognis, 30% |
| Cocamidopropyl Hydroxysultaine | Mirataine CBS, Rhodia |
| Lauryl Glucoside | Plantacare 1200 UP, BASF Cognis, 50% |
| Decyl Glucoside | Plantacare 2000 UP, BASF Cognis |
| Coco Glucoside | Plantacare 818 UP, BASF Cognis, 51% |
| Caprylyl/Capryl Glucoside | Plantacare 810 UP, BASF Cognis |
| Sodium Cocoyl Glutamate | Plantapon ACG HC, BASF Cognis |
| Sodium Lauroyl Glutamate | Amisoft LS-11, Ajinomoto |
| Disodium Cocoyl Glutamate | Planatpon ACG LC, BASF Cognis |
| Sodium Cocoyl Hydrolyzed Wheat Protein Glutamate | PGFAC-S, BASF Cognis |
| Sodium Cocoyl Glycinate | Hostapon SG, Clariant |

(fortgesetzt)

| INCI-Name: | Handelsname: |
|---|---|
| Sodium C14-16 Olefin Sulfonate | Bioterge AS-40 AOS, Stepan |
| Sodium Lauroyl Sarcosinate | Crodasinic LS 30, Croda, 30% |
| Lauroyl Sarcosine | Crodasinic L, Croda |
| Sodium Cocoyl Isethionate | Hostapon STCI-85 P, Clariant |
| Rückfetter / Emollients: | |
| Polyglyceryl-3 Caprate | TEGOSOFT PC 31, Evonik Industries AG, 100% |
| Glyceryl Caprylate | Dermosoft GMCY, Dr. Straetmans |
| Glyceryl Laurate | Imwitor 312, Sasol |
| Diethylhexyl Carbonate | TEGOSOFT DEC, Evonik Industries AG, 100% |
| Caprylic/Capric Triglyceride | TEGOSOFT CT, Evonik Industries AG, 100% |
| PEG-7 Glyceryl Cocoate | TEGOSOFT GC, Evonik Industries AG, 100% |
| Sucrose Cocoate | TEGOSOFT LSE 65 K, Evonik Industries AG, 100% |
| Sorbitol | Sorbitol USP Powder, Lipo |
| Glycerin | Glycerol EP, vegetable, Spiga Nord, 99,7% |
| Dicaprylylether | Cetiol OE, BASF Cognis |
| Schaumbooster: | |
| Hydroxypropyl Methylcellulose | TEGOCEL HPM 50, Evonik Industries AG, 100% |
| Solubilisatoren: | |
| PEG-40 Hydrogenated Castor Oil | TAGAT CH 40, Evonik Industries AG, 100% |
| Pentylene Glycol | Hydrolite-5 616751, Symrise |
| Laureth-2 | Marlipal 24/20, Sasol |
| Polyglyceryl-4 Caprate | TEGOSOFT PC 41, Evonik Industries AG, 100% |

*Anwendungstechnik Homecare:*

Die erfindungsgemäßen Sulfosuccinate eignen sich allgemein für die Anwendung in Reinigungsmitteln für harte Oberflächen und insbesondere in Handgeschirrspülmittel.

In Reinigungsmitteln für harte Oberflächen tragen Amphotenside und besonders Alkylethersulfate primär zur Reinigungswirkung bei. Der Zusatz eines hautmilden anionischen Tensids führt zu einem besseren Schaumverhalten der Gesamtformulierung. Die vorliegende Erfindung betrifft ferner die Verwendung eines erfindungsgemäßen Mittels zur Reinigung harter Oberflächen, insbesondere von Geschirr. Als harte Oberflächen kommen neben Geschirr, Gläser und Besteck auch alle übrigen harten Oberflächen, insbesondere aus Glas, Porzellan, Keramik, polymeren oder metallischen Werkstoffen in Haushalt und Gewerbe in Frage.

Verglichen zu anderen Sulfosuccinaten führt die Verwendung des erfindungsgemäßen Sulfosuccinates zu einem erhöhten Schaumverhalten. Tab. 30 zeigt einen Vergleich zu markterhältlichen Vergleichsprodukten in hartem Wasser (20° dH). Die Austestung erfolgte in einem Auslaufschaumtest mit einer Einsatzmenge von 0,02% Aktivgehalt.

Tab. 30: Schaumverhalten im Auslaufschaumtest mit 0,02% Tensid-Aktivgehalt

| Tensidsystem = 10 : 4,5 : 1,5 | | | | |
|---|---|---|---|---|
| Formulierung | Start | 30 s | 60 s | 120 s |
| SLES : CAPB : REWOPOL® SB CS 50 | 21,0 | 18,0 | 17,5 | 17,0 |
| SLES : CAPB : REWOPOL® SB FA 30 | 20,0 | 17,5 | 16,5 | 16,0 |
| SLES : CAPB : Erf.-gemäßes Beispiel 2 | 26,0 | 21,5 | 21,0 | 20,0 |
| SLES : CAPB : REWOPOL® SB C 212 | 20,0 | 18,0 | 17,0 | 16,5 |
| SLES: Sodium Laureth Sulfate;<br>CAPB: Cocamidopropyl Betaine;<br>REWOPOL® SB CS 50: Disodium PEG-5 Laurylcitrate Sulfosuccinate (and) Sodium Laureth Sulfate;<br>REWOPOL® SB FA 30 B: Disodium Laureth Sulfosuccinate;<br>REWOPOL® SB C 212: Disodium Cocamido MEA-Sulfosuccinate. | | | | |

*Formulierungsbeispiele Home Care:*

Tab. 31: Hautfreundliches Geschirrspülmittel zur manuellen Anwendung

| | |
|---|---|
| Texapon® N70, BASF Cognis, 70%-ig, (INCI: Sodium Laureth Sulfate) | 28,5% |
| Sulfosuccinat Beispiel 2, 60%-ig | 2,5% |
| TEGO® Betain F 50, Evonik Industries AG, 38%-ig | 11,8% |
| Water | 57,2% |
| Parfum, Preservative | q.s. |

Tab. 32: Milder Küchenreiniger

| | |
|---|---|
| Sulfosuccinat Beispiel 3, 60%-ig | 2,5% |
| TEGO® Betain C 60, Evonik Industries AG, 47%-ig | 2,2% |
| Trilon® M, BASF, 40%-ig | 0,7% |
| Water | 94,6% |
| Parfum, Preservative | q.s. |

**Patentansprüche**

1. Auf Polyglycerinpartialestern basierende Sulfosuccinate der allgemeinen Formel I

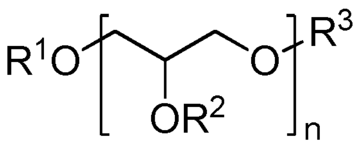

allgemeine Formel I

mit

$R^1$, $R^2$, $R^3$ = unabhängig voneinander gleich oder verschieden H, $R^4$ oder $R^5$,
$R^4$ = gesättigter oder teilweise ungesättigter, 6 - 22 C-Atome enthaltender Acylrest, der durch Hydroxylgruppen

substituiert sein kann, bevorzugt ein Acylrest mit 8 - 18 C-Atomen, insbesondere ein Capryloyl-, Caproyl- oder Lauroylrest, wobei auch Mischungen von Acylresten vorliegen können,
$R^5$ = ausgewählt aus
Sulfobernsteinsäurereste der Formel IIa oder IIb

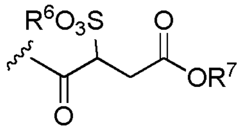 Formel IIa, 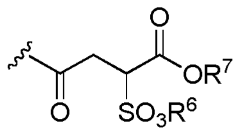 Formel IIb

und Sulfomethylbernsteinsäurereste der Formel IIc oder IId

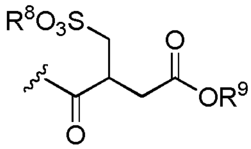 Formel IIc, 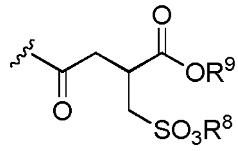 Formel IId,

mit
$R^6$, $R^7$, $R^8$, $R^9$ = unabhängig voneinander gleich oder verschieden H, ein Alkali- oder ½ Erdalkalimetall oder eine Ammoniumgruppe, bevorzugt Natrium,
n = 2 bis 16,

**dadurch gekennzeichnet, dass**
im statistischen Mittel pro Molekül der allgemeinen Formel I 0,2 bis 0,8, bevorzugt 0,3 bis 0,6 Reste $R^4$ und im statistischen Mittel pro Molekül der allgemeinen Formel I 0,3 bis 6, bevorzugt 0,5 bis 5, insbesondere bevorzugt 0,7 bis 3 Reste $R^5$ vorliegen.

**2.** Sulfosuccinate gemäß Anspruch 1, **dadurch gekennzeichnet, dass** sie im statistischen Mittel ein Gewichtsverhältnis von Carbonsäure $R^4OH$ zu Polyglyceringrundgerüst (allgemeine Formel I mit $R^1$, $R^2$, $R^3$ = H) von 0,10 zu 1 bis 0,50 zu 1, insbesondere von 0,15 zu 1 bis 0,40 zu 1 aufweisen.

**3.** Sulfosuccinate gemäß Anspruch 1 oder 2, **dadurch gekennzeichnet, dass** sie einen mittlere Polymerisationsgrad n von 2 bis 11, besonders bevorzugt von 3 bis 8, aufweisen.

**4.** Sulfosuccinate gemäß mindestens einem der Ansprüche 1 bis 3, **dadurch gekennzeichnet, dass**
$R^4$ = ein Acylrest einer Fettsäure ausgewählt aus Önanthsäure, Caprylsäure, Pelargonsäure, Caprinsäure, Laurinsäure, Myristinsäure, Pentadecansäure, Palmitinsäure, Margarinsäure, Stearinsäure, Undecylensäure, Myristoleinsäure, Palmitoleinsäure, Petroselinsäure, Ölsäure, Elaidinsäure, Vaccensäure, Linolsäure, $\alpha$-Linolensäure, $\gamma$-Linolensäure, Calendulasäure, Punicinsäure, $\alpha$-Elaeostearinsäure, $\beta$-Elaeostearinsäure und Mischungen dieser ist, wobei Capryl-, Caprin- oder Laurinsäurereste, insbesondere deren Mischungen, und Kokosfettsäuremischungen besonders bevorzugt sind.

**5.** Sulfosuccinate gemäß mindestens einem der Ansprüche 1 bis 4, **dadurch gekennzeichnet, dass**
$R^5$ ausgewählt ist aus Sulfobernsteinsäureresten der Formel IIa oder IIb.

**6.** Sulfosuccinate gemäß mindestens einem der Ansprüche 1 bis 4, **dadurch gekennzeichnet, dass**
$R^5$ ausgewählt ist aus Sulfomethylbernsteinsäureresten der Formel IIc oder IId.

**7.** Sulfosuccinate gemäß mindestens einem der Ansprüche 1 bis 4, **dadurch gekennzeichnet, dass**
$R^4$ = der Acylrest einer natürlichen Fettsäure ausgewählt aus Caprylsäure, Caprinsäure, Laurinsäure, insbesondere deren Mischungen, und Kokosfettsäuremischungen und
$R^5$ ausgewählt aus Sulfobernsteinsäureresten der Formel IIa oder IIb ist.

**8.** Verfahren zur Herstellung von auf Polyglycerinpartialestern basierenden Sulfosuccinaten umfassend die Verfahrensschritte:

A) Umsetzen von Polyglycerin mit einem Polymerisierungsgrad von 2 bis 16 mit 20 bis 80 Molprozent, bevorzugt mit 30 bis 60 Molprozent, bezogen auf das Polyglycerin, einer oder mehrerer gesättigten oder teilweise ungesättigten, 6 - 22 C-Atome enthaltender Carbonsäuren, die durch Hydroxylgruppen substituiert sein können, bevorzugt einer Carbonsäure mit 8 - 18 C-Atomen, insbesondere mit einer Caprylsäure, Caprinsäure, Laurinsäure, insbesondere deren Mischungen, oder Kokosfettsäuremischungen,
B) ausgewählt aus mindestens einem der Verfahrensschritte

B1) Umsetzen mit 30 - 600, bevorzugt 50 - 500, insbesondere 70 - 300 Molprozent Maleinsäureanhydrid und/oder Itaconsäureanhydrid, bezogen auf das Polyglycerin, und
B2) Umsetzen mit 30 - 600, bevorzugt 50 - 500, insbesondere 70 - 300 Molprozent Itaconsäure, bezogen auf das Polyglycerin,

C) Sulfonierung mit Alkali-, Erdalkali- und/oder Ammoniumsulfit-Salzen wie Natriumsulfit oder Natriumhydrogensulfit und gegebenenfalls
D) Aufreinigung der auf Polyglycerinpartialestern basierenden Sulfosuccinate.

**9.** Verfahren gemäß Anspruch 8, **dadurch gekennzeichnet, dass** in Verfahrensschritt A) das Polyglycerin mit 10 - 50 Gewichtsprozent, bevorzugt mit 15 - 40 Gewichtsprozent, bezogen auf das Polyglycerin, mindestens einer Carbonsäure umgesetzt wird.

**10.** Verfahren gemäß Anspruch 8 oder 9, **dadurch gekennzeichnet, dass** in Verfahrensschritt A) das Polyglycerin einen mittleren Polymerisationsgrad n von 2 - 11, besonders bevorzugt von 3 - 8 aufweist.

**11.** Verfahren gemäß mindestens einem der Ansprüche 8 bis 10, **dadurch gekennzeichnet, dass** in Verfahrensschritt A) Fettsäuren ausgewählt aus Capronsäure, Önanthsäure, Caprylsäure, Pelargonsäure, Caprinsäure, Laurinsäure, Myristinsäure, Pentadecansäure, Palmitinsäure, Margarinsäure, Stearinsäure, Undecylensäure, Myristoleinsäure, Palmitoleinsäure, Petroselinsäure, Ölsäure, Elaidinsäure, Vaccensäure, Linolsäure, $\alpha$-Linolensäure, $\gamma$-Linolensäure, Calendulasäure, Punicinsäure, $\alpha$-Elaeostearinsäure und $\beta$-Elaeostearinsäure und Mischungen dieser,
wobei Capryl-, Caprin- oder Laurinsäure, insbesondere deren Mischungen, und Kokosfettsäuremischungen besonders bevorzugt sind, eingesetzt werden.

**12.** Verfahren gemäß mindestens einem der Ansprüche 8 bis 11, **dadurch gekennzeichnet, dass** in Verfahrensschritt B) Maleinsäureanhydrid oder Itaconsäureanhydrid eingesetzt wird.

**13.** Verfahren gemäß mindestens einem der Ansprüche 8 bis 11 **dadurch gekennzeichnet, dass** Verfahrensschritt B2) mit Verfahrensschritt A) als Eintopfsynthese durchgeführt wird.

**14.** Verfahren gemäß mindestens einem der Ansprüche 8 bis 10, **dadurch gekennzeichnet, dass** in Verfahrensschritt A) mindestens eine Carbonsäure ausgewählt aus Caprylsäure, Caprinsäure, Laurinsäure, insbesondere deren Mischungen, und Kokosfettsäuremischungen und in Verfahrensschritt B) Maleinsäureanhydrid gemäß B1) eingesetzt wird.

**15.** Auf Polyglycerinpartialestern basierende Sulfosuccinate erhältlich nach einem Verfahren gemäß mindestens einem der Ansprüche 8 bis 14.

**16.** Verwendung der Sulfosuccinate gemäß mindestens einem der Ansprüche 1 bis 7 oder 15 zur Herstellung von Pflege- und Reinigungsformulierungen sowie Pflanzenschutzformulierungen.

**17.** Pflege- und Reinigungsformulierung sowie Pflanzenschutzformulierungen enthaltend Sulfosuccinate gemäß mindestens einem der Ansprüche 1 bis 7 oder 15.

**18.** Pflege- und Reinigungsformulierung gemäß Anspruch 17, **dadurch gekennzeichnet, dass** sie im Wesentlichen sulfatfrei und/oder im Wesentlichen polyglycoletherfrei und im Wesentlichen frei von alkoxylierten Verbindungen ist.

## Claims

**1.** Polyglycerol partial ester-based sulphosuccinates of the general formula I

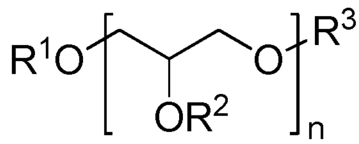

general formula I

where

$R^1$, $R^2$, $R^3$ = independently of one another, identical or different H, $R^4$ or $R^5$,
$R^4$ = a saturated or partially unsaturated acyl radical containing 6 - 22 carbon atoms which can be substituted by hydroxyl groups, preferably an acyl radical having 8 - 18 carbon atoms, in particular a capryloyl, caproyl or lauroyl radical, where mixtures of acyl radicals may also be present,
$R^5$ = selected from sulphosuccinic acid radicals of the formula IIa or IIb

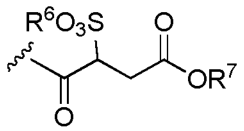

Formula IIa,

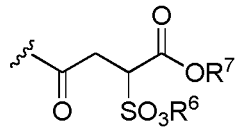

Formula IIb

and sulphomethylsuccinic acid radicals of the formula IIc or IId

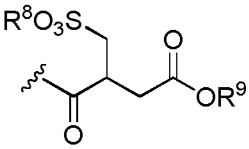

Formula IIc,

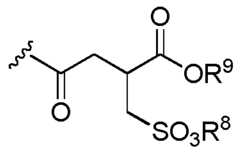

Formula IId,

where
$R^6$, $R^7$, $R^8$, $R^9$ = independently of one another, identical or different H, an alkali metal or ½ alkaline earth metal or an ammonium group, preferably sodium,
n = 2 to 16,

**characterized in that**
on statistical average, per molecule of the general formula I, 0.2 to 0.8, preferably 0.3 to 0.6, radicals $R^4$ and on statistical average, per molecule of the general formula I 0.3 to 6, preferably 0.5 to 5, particularly preferably 0.7 to 3, radicals $R^5$ are present.

2. Sulphosuccinates according to Claim 1, **characterized in that**, on statistical average, they have a weight ratio of carboxylic acid $R^4$ OH to polyglycerol basic backbone (general formula I where $R^1$, $R^2$, $R^3$ = H) of 0.10:1 to 0.50:1, in particular from 0.15:1 to 0.40:1.

3. Sulphosuccinates according to Claim 1 or 2, **characterized in that** they have an average degree of polymerization n of 2 to 11, particularly preferably from 3 to 8.

4. Sulphosuccinates according to at least one of Claims 1 to 3, **characterized in that** $R^4$ = an acyl radical of a fatty acid selected from oenanthic acid, caprylic acid, pelargonic acid, capric acid, lauric acid, myristic acid, pentadecanoic acid, palmitic acid, margaric acid, stearic acid, undecylenic acid, myristoleic acid, palmitoleic acid, petroselic acid, oleic acid, elaidic acid, vaccenic acid, linoleic acid, α-linolenic acid, γ-linolenic acid, calendic acid, punicic acid, α-elaeostearic acid, β-elaeostearic acid and mixtures of these, where caprylic, capric or lauric acid radicals, in particular mixtures thereof, and coconut fatty acid mixtures are particularly preferred.

5. Sulphosuccinates according to at least one of Claims 1 to 4, **characterized in that** $R^5$ is selected from sulphosuccinic acid radicals of the formula IIa or IIb.

**6.** Sulphosuccinates according to at least one of Claims 1 to 4, **characterized in that** $R^5$ is selected from sulphomethylsuccinic acid radicals of the formula IIc or IId.

**7.** Sulphosuccinates according to at least one of Claims 1 to 4, **characterized in that** $R^4$ = the acyl radical of a natural fatty acid selected from caprylic acid, capric acid, lauric acid, in particular mixtures thereof, and coconut fatty acid mixtures and $R^5$ is selected from sulphosuccinic acid radicals of the formula IIa or IIb.

**8.** Process for the preparation of polyglycerol partial ester-based sulphosuccinates comprising the process steps:

A) reaction of polyglycerol having a degree of polymerization of 2 to 16 with 20 to 80 mol per cent, preferably with 30 to 60 mol per cent, based on the polyglycerol, of one or more saturated or partially unsaturated carboxylic acids containing 6 - 22 carbon atoms which may be substituted by hydroxyl groups, preferably a carboxylic acid having 8 - 18 carbon atoms, in particular with a caprylic acid, capric acid, lauric acid, in particular mixtures thereof, or coconut fatty acid mixtures,
B) selected from at least one of the process steps

B1) reaction with 30 - 600, preferably 50 - 500, in particular 70 - 300 mol per cent of maleic anhydride and/or itaconic anhydride, based on the polyglycerol, and
B2) reaction with 30 - 600, preferably 50 - 500, in particular 70 - 300 mol per cent of itaconic acid, based on the polyglycerol,

C) sulphonation with alkali metal, alkaline earth metal and/or ammonium sulphite salts such as sodium sulphite or sodium hydrogensulphite and optionally
D) purification of the sulphosuccinates based on polyglycerol partial esters.

**9.** Process according to Claim 8, **characterized in that**, in process step A), the polyglycerol is reacted with 10 - 50 per cent by weight, preferably with 15 - 40 per cent by weight, based on the polyglycerol, of at least one carboxylic acid.

**10.** Process according to Claim 8 or 9, **characterized in that**, in process step A), the polyglycerol has an average degree of polymerization n of 2 - 11, particularly preferably 3 - 8.

**11.** Process according to at least one of Claims 8 to 10, **characterized in that**, in process step A), fatty acids selected from caproic acid, oenanthic acid, caprylic acid, pelargonic acid, capric acid, lauric acid, myristic acid, pentadecanoic acid, palmitic acid, margaric acid, stearic acid, undecylenic acid, myristoleic acid, palmitoleic acid, petroselic acid, oleic acid, elaidic acid, vaccenic acid, linoleic acid, $\alpha$-linolenic acid, $\gamma$-linolenic acid, calendic acid, punicic acid, $\alpha$-elaeostearic acid and $\beta$-elaeostearic acid and mixtures of these, where caprylic, capric or lauric acid, in particular mixtures thereof, and coconut fatty acid mixtures are particularly preferred, are used.

**12.** Process according to at least one of Claims 8 to 11, **characterized in that**, in process step B), maleic anhydride or itaconic anhydride is used.

**13.** Process according to at least one of Claims 8 to 11, **characterized in that** process step B2) is carried out with process step A) as a one-pot synthesis.

**14.** Process according to at least one of Claims 8 to 10, **characterized in that**, in process step A), at least one carboxylic acid selected from caprylic acid, capric acid, lauric acid, in particular mixtures thereof, and coconut fatty acid mixtures and, in process step B), maleic anhydride according to B1) is used.

**15.** Polyglycerol partial ester-based sulphosuccinates obtainable by a process according to at least one of Claims 8 to 14.

**16.** Use of the sulphosuccinates according to at least one of Claims 1 to 7 or 15 for producing care and cleaning formulations and also crop protection formulations.

**17.** Care and cleaning formulation and also crop protection formulations comprising sulphosuccinates according to at least one of Claims 1 to 7 or 15.

**18.** Care and cleaning formulation according to Claim 17, **characterized in that** it is essentially sulphate-free and/or essentially polyglycol ether-free and essentially free from alkoxylated compounds.

**Revendications**

1. Sulfosuccinates à base d'esters partiels de polyglycérol, de formule générale I

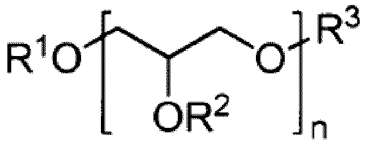

formule générale I

où

$R^1$, $R^2$, $R^3$ = identiques ou différents, chacun indépendamment H, $R^4$ ou $R^5$,
$R^4$ = un radical acyle saturé ou partiellement insaturé, contenant de 6 à 22 atomes de carbone, qui peut être substitué par des groupes hydroxy, de préférence un radical acyle ayant de 8 à 18 atomes de carbone, en particulier un radical capryloyle, caproyle ou lauroyle, des mélanges de radicaux acyle pouvant également être présents,
$R^5$ = choisi parmi
des radicaux sulfosuccinyle de formule IIa ou IIb

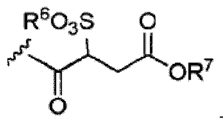 formule IIa, 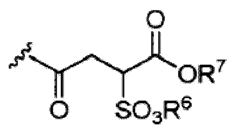 formule IIb

et des radicaux sulfométhylsuccinyle de formule IIc ou IId

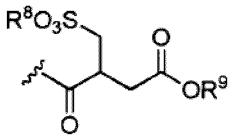 formule IIc, 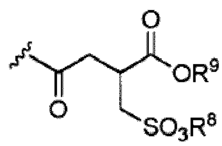 formule IId,

où
$R^6$, $R^7$, $R^8$, $R^9$ = identiques ou différents, indépendamment les uns des autres, H, un atome de métal alcalin ou un ½ atome de métal alcalino-terreux ou un groupe ammonium, de préférence un atome de sodium,
n = 2 à 16,

**caractérisés en ce que**
par molécule de formule générale I est présent en moyenne statistique 0,2 à 0,8, de préférence 0,3 à 0,6 radical $R^4$ et par molécule de formule générale I sont présents en moyenne statistique 0,3 à 6, de préférence 0,5 à 5, de façon particulièrement préférée 0,7 à 3 radicaux $R^5$.

2. Sulfosuccinates selon la revendication 1, **caractérisés en ce qu'**ils présentent en moyenne statistique un rapport pondéral d'acide carboxylique $R^4OH$ à structure de base polyglycérol (formule générale où $R^1$, $R^2$, $R^3$ = H) de 0,10 : 1 à 0,50 : 1, en particulier de 0,15 : 1 à 0,40 : 1.

3. Sulfosuccinates selon la revendication 1 ou 2, **caractérisés en ce qu'**ils présentent un degré moyen de polymérisation n de 2 à 11, de façon particulièrement préférée de 3 à 8.

4. Sulfosuccinates selon au moins l'une quelconque des revendications 1 à 3, caractérisés en ce $R^4$ = un radical acyle d'un acide gras choisi parmi l'acide oenanthique, l'acide caprylique, l'acide pélargonique, l'acide caprique, l'acide laurique, l'acide myristique, l'acide pentadécanoïque, l'acide palmitique, l'acide margarique, l'acide stéarique, l'acide undécylénique, l'acide myristoléique, l'acide palmitoléique, l'acide pétrosélinique, l'acide oléique, l'acide élaïdique,

l'acide vaccénique, l'acide linoléique, l'acide α-linolénique, l'acide γ-linolénique, l'acide calendulaïque, l'acide punicique, l'acide α-éléostéarique, l'acide β-éléostéarique et des mélanges de ceux-ci,
les radicaux d'acides caprylique, caprique ou laurique, en particulier leurs mélanges, ou des mélanges d'acides gras de coco étant particulièrement préférés.

**5.** Sulfosuccinates selon au moins l'une quelconque des revendications 1 à 4, **caractérisés en ce que** $R^5$ est choisi parmi les radicaux sulfosuccinyle de formule IIa ou IIb.

**6.** Sulfosuccinates selon au moins l'une quelconque des revendications 1 à 4, **caractérisés en ce que** $R^5$ est choisi parmi les radicaux sulfométhylsuccinyle de formule IIc ou IId.

**7.** Sulfosuccinates selon au moins l'une quelconque des revendications 1 à 4, **caractérisés en ce que**
$R^4$ = le radical acyle d'un acide gras naturel choisi parmi l'acide caprylique, l'acide caprique, l'acide laurique, en particulier des mélanges de ceux-ci et des mélanges d'acides gras de coco et
$R^5$ est choisi parmi les radicaux sulfosuccinyle de formule IIa ou IIb.

**8.** Procédé pour la préparation de sulfosuccinates à base d'esters partiels de polyglycérol, comprenant les étapes de processus :

A) mise en réaction de polyglycérol, ayant un degré de polymérisation de 2 à 16, avec 20 à 80 pour cent en moles, de préférence avec 30 à 60 pour cent en moles, par rapport au polyglycérol, d'un ou de plusieurs acides carboxyliques saturés ou partiellement insaturés, contenant de 6 à 22 atomes de carbone, qui peuvent être substitués par des groupes hydroxy, de préférence d'un acide carboxylique ayant de 8 à 18 atomes de carbone, en particulier avec un acide caprylique, caprique, laurique, en particulier des mélanges de ceux-ci, ou des mélanges d'acides gras de coco,
B) une étape choisie parmi au moins l'une des étapes de processus

B1) mise en réaction avec 30 - 600, de préférence 50 - 500, en particulier 70 - 300 pour cent en moles d'anhydride maléique et/ou d'anhydride itaconique, par rapport au polyglycérol, et
B2) mise en réaction avec 30 - 600, de préférence 50 - 500, en particulier 70 - 300 pour cent en moles d'acide itaconique, par rapport au polyglycérol,

C) sulfonation avec des sels sulfites de métaux alcalins, de métaux alcalino-terreux et/ou d'ammonium, tels que le sulfite de sodium ou l'hydrogénosulfite de sodium et éventuellement
D) purification des sulfosuccinates à base d'esters partiels de polyglycérol.

**9.** Procédé selon la revendication 8, **caractérisé en ce que** dans l'étape A) du processus on fait réagir le polyglycérol avec 10 - 50 pour cent en poids, de préférence avec 15 - 40 pour cent en poids, par rapport au polyglycérol, d'au moins un acide carboxylique.

**10.** Procédé selon la revendication 8 ou 9, **caractérisé en ce que** dans l'étape A) du processus le polyglycérol présente un degré moyen de polymérisation de 2 à 11, de façon particulièrement préférée de 3 à 8.

**11.** Procédé selon au moins l'une quelconque des revendications 8 à 10, **caractérisé en ce que** dans l'étape A) du processus on utilise des acides gras choisis parmi l'acide caproïque, l'acide oenanthique, l'acide caprylique, l'acide pélargonique, l'acide caprique, l'acide laurique, l'acide myristique, l'acide pentadécanoïque, l'acide palmitique, l'acide margarique, l'acide stéarique, l'acide undécylénique, l'acide myristoléique, l'acide palmitoléique, l'acide pétrosélinique, l'acide oléique, l'acide élaïdique, l'acide vaccénique, l'acide linoléique, l'acide α-linolénique, l'acide γ-linolénique, l'acide calendulaïque, l'acide punicique, l'acide α-éléostéarique et l'acide β-éléostéarique et des mélanges de ceux-ci, l'acide caprylique, l'acide caprique ou l'acide laurique, en particulier leurs mélanges, et des mélanges d'acides gras de coco étant particulièrement préférés.

**12.** Procédé selon au moins l'une quelconque des revendications 8 à 11, **caractérisé en ce que** dans l'étape B) du processus on utilise l'anhydride maléique ou l'anhydride itaconique.

**13.** Procédé selon au moins l'une quelconque des revendications 8 à 11, **caractérisé en ce qu'**on effectue l'étape B2) du processus avec l'étape de processus A) en tant que synthèse en un seul récipient.

**14.** Procédé selon au moins l'une quelconque des revendications 8 à 10, **caractérisé en ce que** dans l'étape A) du processus on utilise au moins un acide carboxylique choisi parmi l'acide caprylique, l'acide caprique, l'acide laurique, en particulier leurs mélanges et des mélanges d'acides gras de coco, et dans l'étape B) du processus on utilise l'anhydride maléique selon B1).

**15.** Sulfosuccinates à base d'esters partiels de polyglycérol, pouvant être obtenus conformément à un procédé selon au moins l'une quelconque des revendications 8 à 14.

**16.** Utilisation des sulfosuccinates selon au moins l'une quelconque des revendications 1 à 7 ou 15, pour la fabrication de compositions de soin et de nettoyage ainsi que de compositions phytosanitaires.

**17.** Composition de soin et de nettoyage ainsi que compositions phytosanitaires, contenant des sulfosuccinates selon au moins l'une quelconque des revendications 1 à 7 ou 15.

**18.** Composition de soin et de nettoyage selon la revendication 17, **caractérisé en ce qu'**elle est pratiquement exempte de sulfates et/ou pratiquement exempte de polyglycoléthers et pratiquement exempte de composés alcoxylés.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 1992018470 A
- DE 102005012479
- EP 0794173 A
- SU 1330130
- DE OS2700072 A
- EP 2273966 A1
- WO 2007115872 A


### In der Beschreibung aufgeführte Nicht-Patentliteratur


- **CASSEL et al.** Original synthesis of linear, branched and cyclic oligoglycerol Standards. *J. Org. Chem.,* 2001, 875-896
- **G. JAKOBSON.** *Fette, Seifen, Anstrichmittel,* 1986, vol. 88, 101-105
- **BEISPIEL K. SCHRADER.** Grundlagen und Rezepturen der Kosmetika. Hüthig Buch Verlag Heidelberg, 329-341
- **W.J. W. PAPE ; U. PFANNENBECKER ; U. HOPPE.** *Mol. Toxicol.,* 1987, vol. 1, 525